# EUROPEAN PATENT SPECIFICATION

(11) **EP 0 665 886 B1**
(45) Date of publication and mention of the grant of the patent: **11.06.2003**
(21) Application number: 93924946.2
(22) Date of filing: 18.10.1993
(51) Int. Cl.: C12N 15/12, C12N 15/54, C12N 9/12, C12Q 1/68, G01N 33/577

(54) **CYCLIN COMPLEX REARRANGEMENT AND USES RELATED THERETO**
UMLAGERUNG DES CYCLIN KOMLEXES UND SEINE DARAUF BEZOGENEN ANWENDUNGEN
REAGENCEMENT DE COMPLEXES DE CYCLINES ET LEURS UTILISATIONS

(30) Priority: 16.10.1992 US 963308; 17.12.1992 US 991997
(43) Date of publication of application: 09.08.1995
(73) Proprietor: COLD SPRING HARBOR LABORATORY, Cold Spring Harbor, NY 11724 (US)
(72) Inventor: BEACH, David, H., Huntington Bay, NY 11743 (US); XIONG, Yue, Huntington Station, NY 11746 (US)
(74) Representative: Horner, Martin Grenville
(86) International application number: US9309945
(87) International publication number: WO94009135

(56) References cited:
- EMBO JOURNAL vol. 11, no. 8 , August 1992 , IRL PRESS LIM., OXFORD, ENGL.; pages 2909 - 2917 M. MEYERSON ET AL. 'A family of human cdc2-related protein kinases'
- TRENDS IN BIOCHEMICAL SCIENCE vol. 17, no. 8 , August 1992 , ELSEVIER SCIENCE, AMSTERDAM, NL; pages 281 - 285 M. KIRSCHER 'The cell cycle then and now'
- AMERICAN JOURNAL OF PATHOLOGY vol. 134, no. 4 , April 1989 , AM. J. PATHOLOGIST, HAGERSTOWN, MD, US; pages 733 - 739 R.L. GARCIA ET AL. 'Analysis of proliferative grade using anti-PCNA/Cyclin monoclonal antobodies in fixed, embeded tissues'
- CELL vol. 71, no. 3 , 30 October 1992 , CELL PRESS, CAMBRIDGE, MA,US; pages 505 - 514 Y. XIONG ET AL. 'D type cyclins associate with multiple protein kinases and the DNA replication and repair factor PCNA'
- GENES & DEVELOPMENT vol. 7, no. 8 , August 1993 , CSH LABORATORY PRESS, CSH, N.Y., US; pages 1572 - 1583 Y. XIONG ET AL. 'Subunit rearrangement of the cyclin-dependent kinases is associated with cellular transformation'
- MOLECULAR BIOLOGY OF THE CELL vol. 4, no. 9 , September 1993 , AMERICAN SOCIETY FOR CELL BIOLOGY, pages 897 - 906 H. ZHANG ET AL. 'Proliferating cell nuclear antigen and p21 are components of multiple cell cycle kinase complexes'
- NATURE vol. 366 , 16 December 1993 , MACMILLAN JOURNALS LTD., LONDON, UK; pages 704 - 707 M. SERRANO ET AL. 'A new regulatory motif in cell-cycle control causing specific inhibition of cyclin D/CDK4'

### SEQUENCE LISTING

(1) GENERAL INFORMATION:
   (i) APPLICANT:
      (A) NAME: Cold Spring Harbor Laboratory
      (B) STREET: 100 Bungtown Road
      (C) CITY: Cold Spring Harbor
      (D) STATE: NY
      (E) COUNTRY: USA
      (F) POSTAL CODE (ZIP): 11724
      (G) TELEPHONE: (617) 227-7400
      (H) TELEFAX: (617) 227-5941
   (ii) TITLE OF INVENTION: Cyclin Complex Rearrangement and Uses Related Thereto
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII(text)
   (vi) PRIOR APPLICATION DATA:
      (A) APPLICATION NUMBER: US 07/963,308
      (B) FILING DATE: 16-OCT-1992
   (vi) PRIOR APPLICATION DATA:
      (A) APPLICATION NUMBER; US 07/991,997
      (B) FILING DATE: 17-DEC-1992
(2) INFORMATION FOR SEQ ID NO:1:
   (i) SEQUENCE CHARACTERISTICS:
      (A) LENGTH: 1089 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 13..888
   (xi) SEQUENCE DESCRIPTION: SEQ ID NO:1:
   CCGGCCGCCG CG ATG CAG AAA TAC GAG AAA CTG GAA AAG ATT GGG GAA        48
                 Met Gln Lys Tyr Glu Lys Leu Glu Lys Ile Gly Glu
                     1                 5                   10
   GGC ACC TAC GGA ACT GTG TTC AAG GCC AAA AAC CGG GAG ACT CAT GAG    96
   Gly Thr Tyr Gly Thr Val Phe Lys Ala Lys Asn Arg Glu Thr His Glu
            15                  20                  25
   ATC GTG GCT CTA AAA CGG GTG AGG CTG GAT GAC GAT GAT GAG GGT GTG    144
   Ile Val Ala Leu Lys Arg Val Arg Leu Asp Asp Asp Asp Glu Gly Val
        30                  35                  40
   CCG AGT TCC GCC CTC CGG GAG ATC TGC CTA CTC AAG GAG CTG AAG CAC    192
   Pro Ser Ser Ala Leu Arg Glu Ile Cys Leu Leu Lys Glu Leu Lys His
    45                  50                  55                  60
   AAG AAC ATC GTC AGG CTT CAT GAC GTC CTG CAC AGC GAC AAG AAG CTG    240
   Lys Asn Ile Val Arg Leu His Asp Val Leu His Ser Asp Lys Lys Leu
                    65                  70                  75
   ACT TTG GTT TTT GAA TTC TGT GAC CAG GAC CTG AAG AAG TAT TTT GAC    288
   Thr Leu Val Phe Glu Phe Cys Asp Gln Asp Leu Lys Lys Tyr Phe Asp
                80                  85                  90
   AGT TGC AAT GGT GAC CTC GAT CCT GAG ATT GTA AAG TCA TTC CTC TTC    336
   Ser Cys Asn Gly Asp Leu Asp Pro Glu Ile Val Lys Ser Phe Leu Phe
            95                 100                 105
   CAG CTA CTA AAA GGG CTG GGA TTC TGT CAT AGC CGC AAT GTG CTA CAC    384
   Gln Leu Leu Lys Gly Leu Gly Phe Cys His Ser Arg Asn Val Leu His
       110                 115                 120
   AGG GAC CTG AAG CCC CAG AAC CTG CTA ATA AAC AGG AAT GGG GAG CTG    432
   Arg Asp Leu Lys Pro Gln Asn Leu Leu Ile Asn Arg Asn Gly Glu Leu
   125                 130                 135                 140
   AAA TTG GCT GAT TTT GGC CTG GCT CGA GCC TTT GGG ATT CCC GTC CGC    480
   Lys Leu Ala Asp Phe Gly Leu Ala Arg Ala Phe Gly Ile Pro Val Arg
                   145                 150                 155
   TGT TAC TCA GCT GAG GTG GTC ACA CTG TGG TAC CGC CCA CCG GAT GTC    528
   Cys Tyr Ser Ala Glu Val Val Thr Leu Trp Tyr Arg Pro Pro Asp Val
               160                 165                 170
   CTC TTT GGG GCC AAG CTG TAC TCC ACG TCC ATC GAC ATG TGG TCA GCC    576
   Leu Phe Gly Ala Lys Leu Tyr Ser Thr Ser Ile Asp Met Trp Ser Ala
           175                 180                 185
   GGC TGC ATC TTT GCA GAG CTG GCC AAT GCT GGG CGG CCT CTT TTT CCC    624
   Gly Cys Ile Phe Ala Glu Leu Ala Asn Ala Gly Arg Pro Leu Phe Pro
       190                 195                 200
   GGC AAT GAT GTC GAT GAC CAG TTG AAG AGG ATC TTC CGA CTG CTG GGG    672
   Gly Asn Asp Val Asp Asp Gln Leu Lys Arg Ile Phe Arg Leu Leu Gly
   205                 210                 215                 220
   ACG CCC ACC GAG GAG CAG TGG CCC TCT ATG ACC AAG CTG CCA GAC TAT     720
   Thr Pro Thr Glu Glu Gln Trp Pro Ser Met Thr Lys Leu Pro Asp Tyr
                   225                     230                 235
   AAG CCC TAT CCG ATG TAC CCG GCC ACA ACA TCC CTG GTG AAC GTC GTG     768
   Lys Pro Tyr Pro Met Tyr Pro Ala Thr Thr Ser Leu Val Asn Val Val
               240                     245                 250
   CCC AAA CTC AAT GCC ACA GGG AGG GAT CTG CTG CAG AAC CTT CTG AAG     816
   Pro Lys Leu Asn Ala Thr Gly Arg Asp Leu Leu Gln Asn Leu Leu Lys
           255                     260                 265
   TGT AAC CCT GTC CAG CGT ATC TCA GCA GAA GAG GCC CTG CAG CAC CCC     864
   Cys Asn Pro Val Gln Arg Ile Ser Ala Glu Glu Ala Leu Gln His Pro
       270                 275                     280
   TAC TTC TCC GAC TTC TGT CCG CCC TAGGCCCGGG ACCCCCGGCC TCAGCTGGGC   918
   Tyr Phe Ser Asp Phe Cys Pro Pro
   285                 290
   CTGGCCTATT TAAGCCCCTC TTGAGAGGGG TGAGACAGTG GGGGTGCCTG GTGCGCTGTG  978
   CTCAGCAGTG CTGGGCCAGC CGGGGTGGGG TGCCTGAGCC CGAATTTCTC ACTCCCTTTG 1038
   TGGACTTTAT TTAATTTCAT AAATTGGCTC CTTTCCCACA AAAAAAAAAG G          1089
(2) INFORMATION FOR SEQ ID NO:2:
   (i) SEQUENCE CHARACTERISTICS:
      (A) LENGTH: 292 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gln Lys Tyr Glu Lys Leu Glu Lys Ile Gly Glu Gly Thr Tyr Gly
    1               5                  10                  15
   Thr Val Phe Lys Ala Lys Asn Arg Glu Thr His Glu Ile Val Ala Leu
               20                  25                  30
   Lys Arg Val Arg Leu Asp Asp Asp Asp Glu Gly Val Pro Ser Ser Ala
           35                  40                  45
   Leu Arg Glu Ile Cys Leu Leu Lys Glu Leu Lys His Lys Asn Ile Val
       50                  55                  60
   Arg Leu His Asp Val Leu His Ser Asp Lys Lys Leu Thr Leu Val Phe
   65                  70                  75                  80
   Glu Phe Cys Asp Gln Asp Leu Lys Lys Tyr Phe Asp Ser Cys Asn Gly
                           85                   90                   95
   Asp Leu Asp Pro Glu Ile Val Lys Ser Phe Leu Phe Gln Leu Leu Lys
                   100                 105                 110
   Gly Leu Gly Phe Cys His Ser Arg Asn Val Leu His Arg Asp Leu Lys
               115                 120                 125
   Pro Gln Asn Leu Leu Ile Asn Arg Asn Gly Glu Leu Lys Leu Ala Asp
       130                 135                 140
   Phe Gly Leu Ala Arg Ala Phe Gly Ile Pro Val Arg Cys Tyr Ser Ala
   145                 150                 155                 160
   Glu Val Val Thr Leu Trp Tyr Arg Pro Pro Asp Val Leu Phe Gly Ala
                   165                 170                 175
   Lys Leu Tyr Ser Thr Ser Ile Asp Met Trp Ser Ala Gly Cys Ile Phe
               180                 185                 190
   Ala Glu Leu Ala Asn Ala Gly Arg Pro Leu Phe Pro Gly Asn Asp Val
           195                 200                 205
   Asp Asp Gln Leu Lys Arg Ile Phe Arg Leu Leu Gly Thr Pro Thr Glu
       210                 215                 220
   Glu Gln Trp Pro Ser Met Thr Lys Leu Pro Asp Tyr Lys Pro Tyr Pro
   225                 230                 235                 240
   Met Tyr Pro Ala Thr Thr Ser Leu Val Asn Val Val Pro Lys Leu Asn
                   245                 250                 255
   Ala Thr Gly Arg Asp Leu Leu Gln Asn Leu Leu Lys Cys Asn Pro Val
               260                 265                 270
   Gln Arg Ile Ser Ala Glu Glu Ala Leu Gln His Pro Tyr Phe Ser Asp
           275                 280                 285
   Phe Cys Pro Pro
       290
(2) INFORMATION FOR SEQ ID NO:3:
   (i) SEQUENCE CHARACTERISTICS:
      (A) LENGTH: 948 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 19..465
   (xi) SEQUENCE DESCRIPTION: SEQ ID NO:3:
   AATTCGGCAC GAGGCAGC ATG GAG CCT TCG GCT GAC TGG CTG GCC ACG GCC          51
   GCG GCC CGG GGT CGG GTA GAG GAG GTG CGG GCG CTG CTG GAG GCG GTG          99
   GCG CTG CCC AAC GCA CCG AAT AGT TAC GGT CGG AGG CCG ATC CAG GTC         147
   ATG ATG ATG GGC AGC GCC CGA GTG GCG GAG CTG CTG CTG CTC CAC GGC         195
   GCG GAG CCC AAC TGC GCC GAC CCC GCC ACT CTC ACC CGA CCC GTG CAC         243
   GAC GCT GCC CGG GAG GGC TTC CTG GAC ACG CTG GTG GTG CTG CAC CGG         291
   GCC GGG GCG CGG CTG GAC GTG CGC GAT GCC TGG GGC CGT CTG CCC GTG         339
   GAC CTG GCT GAG GAG CTG GGC CAT CGC GAT GTC GCA CGG TAC CTG CGC         387
   GCG GCT GCG GGG GGC ACC AGA GGC AGT AAC CAT GCC CGC ATA GAT GCC         435
   GCG GAA GGT CCC TCA GAC ATC CCC GAT TGAAAGAACC AGAGAGGCTC               482
   TGAGAAACCT CGGGAAACTT AGATCATCAG TCACCGAAGG TCCTACAGGG CCACAACTGC        542
   CCCCGCCACA ACCCACCCCG CTTTCGTAGT TTTCATTTAG AAAATAGAGC TTTTAAAAAT        602
   GTCCTGCCTT TTAACGTAGA TATAAGCCTT CCCCCACTAC CGTAAATGTC CATTTATATC        662
   ATTTTTTATA TATTCTTATA AAAATGTAAA AAAGAAAAAC ACCGCTTCTG CCTTTTCACT        722
   GTGTTGGAGT TTTCTGGAGT GAGCACTCAC GCCCTAAGCG CACATTCATG TGGGCATTTC    782
   TTGCGAGCCT CGCAGCCTCC GGAAGCTGTC GACTTCATGA CAAGCATTTT GTGAACTAGG    842
   GAAGCTCAGG GGGGTTACTG GCTTCTCTTG AGTCACACTG CTAGCAAATG GCAGAACCAA    902
   AGCTCAAATA AAAATAAAAT TATTTTCATT CATTCACTCA AAAAAA                   948
(2) INFORMATION FOR SEQ ID NO:4:
   (i) SEQUENCE CHARACTERISTICS:
      (A) LENGTH: 148 amino acids
      (B) TYPE: amino acid
      (D) TOPOLOGY: linear
   (ii) MOLECULE TYPE: protein
   (xi) SEQUENCE DESCRIPTION: SEQ ID NO:4:

## Description

### Background of the Invention

Neoplasia is characterized by deregulated cell growth and division. Inevitably, molecular pathways controlling cell growth must interact with those regulating cell division. It was not until very recently, however, that experimental evidence became available to bring such connection to light. Cyclin A was found in association with the adenovirus oncoprotein E1A in virally transformed cells (Giordona et al. *Cell* 58:981 (1989); and Pines et al. *Nature* 346:760 (1990)). In an early hepatocellular carcinoma, the human cyclin A gene was found to be the integration site of a fragment of the hepatitis B virus, which leads to activation of cyclin A transcription and a chimeric viral cyclin A protein that is not degradable *in vitro* (Wang et al. *Nature* 343:555 (1990)). The cell cycle gene implicated most strongly in oncogenesis thus far is the human cyclin D1. It was originally isolated through genetic complementation of yeast G₁ cyclin deficient strains (Xiong et al. *Cell* 65:691(1991); and Lew et al. *Cell* 66:1197 (1991)), as cellular genes whose transcription is stimulated by CSF-1 in murine macrophages (Matsushine et al. *Cell* 65:701 (1991)) and in the putative oncogene *PRADI* rearranged in parathyroid tumors (Montokura et al. *Nature* 350:512 (1991). Two additional human D-type cyclins, cyclins D2 and D3, were subsequently identified using PCR and low-stringency hybridiazation techniques (Inaba et al. *Genomics* 13:565 (1992); and Xiong et al. *Genomics* 13:575 (1992)). Cyclin D1 is genetically linked to the *bcl-1* oncogene, a locus activated by translocation to an immunoglobulin gene enhancer in some B-cell lymphomas and leukemias, and located at a site of gene amplification in 15-20% of human breast cancers and 25-48% of squamous cell cancers of head and neck origin.

Cyclins are proteins that were discovered due to their intense synthesis following the fertilization of marine invertebrate eggs (Rosenthal, E.T. *et al., Cell* 20:487-494 (1980)). It was subsequently observed that the abundance of two types of cyclin, A and B, oscillated during the early cleavage divisions due to abrupt proteolytic degradation of the polypeptides at mitosis and thus, they derived their name (Evans, T. *et al., Cell 33*:389-396 (1983); Swenson, K.I. *et al.*, *Cell 47*:867-870 (1986); Standart, N. *et al., Dev. Biol. 124*:248-258 (1987)). Subsequently, cyclin genes have been isolated from virtually all eukaryotic species and constitute a multigene family (for review, see Xiong et al. *Curr Biology* 1:362 (1991).

Active rather than passive involvement of cyclins in regulation of cell division became apparent with the observation that a clam cyclin mRNA could cause activation of frog oocytes and entry of these cells into M phase (Swenson, K.I. *et al., Cell 7*:867-870 (1986)). Activation of frog oocytes is associated with elaboration of an M phase inducing factor known as MPF (Masui, Y. and C.L. Markert, *J. Exp. Zool. 177*:129-146 (1971); Smith, L.D. and R.E. Ecker, *Dev. Biol. 25*:232-247 (1971)). MPF is a protein kinase in which the catalytic subunit is the frog homolog of the cdc2 protein kinase (Dunphy, W.G. *et al., Cell 54*:423-431 (1988); Gautier, J. *et al., Cell 54*:433-439 (1988); Anon, D. *et al., Cell 55:371*-378 (1988)).

Among the cyclins identified to date, the B-type cyclin has been shown to act in mitosis by serving as an integral subunit of the cdc2 protein kinase (Booher, R. and D. Beach, *EMBO J*. *6*:3441-3447 (1987); Draetta, G. et *al., Cell 56*:829-838 (1989); Labbe, J.C. *et al., Cell 57*:253-263 (1989); Labbe, J.C. *et al., EMBO J. 8*:3053-3058 (1989); Meier, L. *et al., EMBO J. 8*:2275-2282 (1989); Gautier, J. *et al., Cell 60*:487-494 (1990)). The A-type cyclin also independently associates with the cdc2 kinase, forming an enzyme that appears to act earlier in the division cycle than mitosis (Draetta, G. *et al., Cell 56*:829-838 (1989); Minshull, J. *et al., EMBO J. 9*:2865-2875 (1990); Giordano, A. *et al., Cell 58*:981-990 (1989); Pines, J. and T. Hunter, *Nature 346*:760-763 (1990)). Cellular and molecular studies of cyclins in invertebrate and vertebrate embryos have been accompanied by genetic studies, particularly in ascomycete yeasts. In the fission yeast, the cdc13 gene encodes a B-type cyclin that acts in cooperation with cdc2 to regulate entry into mitosis (Booher, R. and D. Beach, *EMBO J., 6*:3441-3447 (1987); Booher, R. and D. Beach, *EMBO J. 7*:2321-2327 (1988); Hagan, I. *et al., J. Cell Sci. 91*:587-595 (1988); Solomon, M., *Cell 54*:738-740 (1988); Goebl, M. and B. Byers, *Cell 54*:433-439 (1988); Booher, R.N. *et al., Cell 58*:485-497 (1989)). Genetic studies in both the budding yeast and fission yeast have revealed that cdc2 (or CDC28 in budding yeast) acts at two independent points in the cell cycle: mitosis and the so-called cell cycle "start" (Hartwell, L.H., *J. Mol. Biol., 104*:803-817 (1971); Nurse, P. and Y. Bissett, *Nature 292*:558-560 (1981); Piggot, J.R *et al., Nature 298*:391-393 (1982); Reed, S.I. and C. Wittenberg, *Proc. Nat. Acad. Sci. USA 87*:5697-5701 (1990)). In budding yeast, the start function of the CDC28 protein also requires association of the catalytic subunit of the protein kinase with ancillary proteins that are structurally related to A and B-type cyclins. This third class of cyclin has been called the CLN class, and three genes comprising a partially redundant gene family have been described (Nash, R. *et al., EMBO J. 7*:4335-4346 (1988); Hadwiger, J.A. *et al., Proc. Natl. Acad. Sci. USA 86*:62556259 (1989); Richardson, H.E. *et al., Cell 59*:1127-1133 (1989)). The CLN genes are essential for execution of start and in their absence, cells become arrested in the G1 phase of the cell cycle. The CLN1 and CLN2 transcripts oscillate in abundance through the cell cycle, but the CLN3 transcript does not. In addition, the CLN2 protein has been shown to oscillate in parallel with its mRNA (Nash, R. *et al., EMBO J. 7*:4335-4346 (1988); Cross, F.R., *Mol. Cell. Biol. 8*:4675-4684 (1988); Richardson, H.E. *et al., Cell 59*:1127-1133 (1988); Wittenberg, *et al.*, 1990)). Although the precise biochemical properties conferred on cdc2/CDC28 by association with different cyclins have not been fully elaborated, genetic studies of cyclin mutants clearly establishes that they confer "G1" and "G2" properties on the catalytic subunit (Bopher, R. and D. Beach, *EMBO J.* 6:3441-3447 (1987); Nash, R. *et al., EMBO J.* 7:4335-4346 (1988); Richardson, H.E. *et al., Cell* 56:1127-1133 (1989)).

cdc2 and cyclins have been found not only in embryos and yeasts; but also in somatic human cells. The function of the cdc2/cyclin B enzyme appears to be the same in human cells as in other cell types (Riabowol, K. *et al., Cell* 57:393-401 (1989)). A human A type cyclin has also been found in association with cdc2. No CLN type cyclin has yet been described in mammalian cells. Abetter understanding of the elements involved in cell cycle regulation and of their interactions would contribute to a better understanding of cell replication and perhaps even alter or control the process.

### Summary of the Invention

The present invention provides a method of identifying a transformed or abnormally proliferating cell comprising:
(A) determining, in a test cell(s), the subunit composition of a complex comprising a protein having a molecular weight of about 16 kDa, 19 kDa or 21 kDa and either a cyclin dependent kinase (cdk), a cyclin or both a cdk and a cyclin, wherein the protein is predominantly a protein having an apparent molecular weight of 21 kDa in a normal cell or is predominantly a protein having an apparent molecular weight of 19 kDa or about 16 kDa in a transformed or abnormally proliferating cell, and
(B) comparing the subunit composition of step (A) with the subunit composition of a like complex found in a normal cell,
wherein an alteration in the subunit composition is indicative of transformation of the test cell(s).

In a further aspect there is provided a purified and/or recombinant polypeptide comprising an amino acid sequence of a polypeptide having an apparent molecular mass of about 15 kDa to 17 kDa, wherein said polypeptide inhibits a cyclin dependent kinase and is encoded by a nucleic acid which specifically hybridizes to the nucleic acid sequence of SEQ ID No. 3.

In a still further aspect there is provided an isolated nucleic acid (sense or antisense) which specifically hybridizes to the nucleic acid sequence of SEQ ID No. 3 and selectively detects a gene encoding a 16 kDa protein which binds to cyclin dependent kinase 4 (cdk4).

There is also provided a diagnostic test kit for identifying transformed cells comprising:
(a) a nucleic acid probe (sense or antisense) which specifically hybridizes to the nucleic acid sequence of SEQ ID No. 3 and selectively detects a nucleic acid encoding a 16 kDa protein which binds to cyclin dependent kinase 4 (cdk4), which probe is for measuring, in a sample of cells isolated from a patient, the presence or absence of a nucleic acid encoding said 16 kDa protein; and/or
(b) an antibody specific for the 16 kDa protein for measuring, in a sample of cells isolated from a patient, an amount of said protein.

In a further aspect there is provided an assay method for identifying an agent which alters the subunit composition of a cyclin-dependent kinase complex, comprising
i. providing a cyclin-dependent kinase (cdk) and the polypeptide of any claims 10 to 15, under conditions wherein the cdk and said 16 kDa polypeptide can form a complex;
ii. contacting the mixture with a candidate agent;
iii. detecting an amount of polypeptide as part of the complex formed;
iv. comparing the amount of polypeptide present in the complex in the presence of the candidate agent to the amount of polypeptide present in the complex in the absence of the candidate agent,
wherein a change in the amount of polypeptide present in the complex in the presence of the candidate agent, relative to the absence of candidate agent, is indicative of an agent which alters the subunit composition of a cyclin-dependent kinase complex.

There is also provided a method for detecting transformation of a mammalian cell by detecting altered expression of a gene encoding a protein having a molecular weight of 15 kDa to 17 kDa which is an inhibitor of cdk4, which method comprises determining, in a test sample of mammalian cells, the presence or absence of nucleic acid which specifically hybridizes to the nucleic acid of SEQ ID No. 3, the presence of said nucleic acid indicating transformation of the cells.

The present invention relates to uses for the novel class of cyclins, referred to as D-type cyclins, which are of mammalian origin and are a new family of cyclins related to, but distinct from, previously described A, B or CLN type cyclins. In particular, it relates to human cyclins, encoded by genes shown to be able to replace a CLN-type gene essential for cell cycle start in yeast, which complement a deficiency of a protein essential for cell cycle start and which, on the basis of protein structure, are on a different branch of the evolutionary tree from A, B or CLN type cyclins. D-type cyclins have been shown to associate, in eukaryotic cells, particularly human cells, with multiple cyclin dependent kinases. They have also been shown to co-precipitate with three polypeptides: a cyclin-dependent kinase, a well characterized DNA replication and repair factor (i.e., proliferating cell nuclear antigen or PCNA) and a polypeptide of 21 kDa apparent molecular weight. Results suggest that D-type cyclin, CDK, PCNA, and p21 exist in a quaternary complex, that many combinatorial variations of the components (e.g., cyclin D1 or D3 and CDK2, CDK4 and CDK5) assemble *in vivo* and that each of the quaternary complexes may have a subtly different role in the cell cycle or in different cell types. In addition, it has been discovered that cellular transformation by DNA tumor viruses, such as SV40, is associated with selective subunit rearrangement of the cyclin D complexes. The association between cyclin D, PCNA, CDKs (including CDK2, CDK4 and CDK5) and p21 is disrupted by introduction of a DNA tumor virus or its oncogenic gene product into mammalian cells. Specifically, as described herein, it has been shown that the association between cyclin D and PCNA, CDKs (CDK2, CDK4 and CDK5) and p21 is disrupted upon introduction of SV40 tumor virus or its oncogenic gene product large T antigen into human diploid cells (as exemplified by normal human diploid fibroblasts). After disassociating from cyclin D and p21, CDK4 becomes associated with a novel polypeptide of 16 kDa (p16). Similarly, cyclin A complexes also undergo subunit rearrangement. After SV40 transformation, p21 association with cyclin A is decreased or completely disassociated. Cyclin A then appears in a complex with a 19 kDA polypeptide (p19).

Thus, it is now known that p21 is associated with cyclin kinases only in normal, untransformed cells, and p16, p19 and potentially other related proteins appear to the cell cycle regulator present in transformed cells. This knowledge serves as the basis for a variety of approaches to modulating cell division by altering the activity (directly or indirectly) of cyclins. It offers specificity in modulating cell division (i.e., the ability to selectively alter cell division in particular cell types or at a particular point in the cycle) because of the specificity of expression of cyclins in cells and the number of possible combinations of the components of the quaternary complex which appear to be formed by cyclins, CDK, PCNA and p21. In a particular embodiment, it offers a means by which cell division can be non-specifically altered by interfering with a common component of the quaternary complex of which D-type cyclin or A-type cyclin is a constituent, such as by interfering with PCNA.

For example, in one embodiment of a therapeutic method of the present invention, formation of the quaternary complex described above is prevented or enhanced or the activity of a complex member is altered as an approach to altering cell division. Here, agents which act indirectly or directly to prevent or enhance complex formation or to alter a constituent's activity can be used. For example, as described above, catalytic activity can be inhibited by preventing activation of the protein kinase. Alternatively, PCNA inhibitors can be introduced into cells in which cell cycle start is to be inhibited, resulting in inhibition of cell division. PCNA inhibitors can act indirectly (e.g., to reduce production of PCNA by interfering with transcription or translation) or directly (e.g., to bind PCNA and prevent it from joining with other complex members). Inhibitors of p21 can also be introduced into cells and interfere, indirectly or directly, with p21 function and/or binding to the complex members. Protein-protein interactions (between or among complex components) can also be altered (reduced or enhanced) to have the desired effect on the cell cycle (to reduce or increase cell division). Agents which block such protein-protein interactions can be used. These include low molecular weight inhibitors, agents which bind to complex components (e.g., antibodies) and agents which degrade or otherwise destroy a component's ability to form a complex with the other proteins. If enhanced quaternary complex formation is desired, agents which increase the ability of complex members to interact and bind (e.g., agents which change the configuration of a complex component so that it is more available for protein-protein interactions necessary for complex formation can be introduced into cells). Enhanced complex formation can also be brought about by increasing in cells the number, activity or availability of the limiting member(s) of the quaternary complex, thus enhancing the rate at which it is formed and its availability to act.

In addition, the subject invention pertains to methods of diagnosing transformation of a cell. Reagents, such as monoclonal antibodies, can be developed that recognize the interactions between the CDKs, cyclins, PCNA and the low molecular weight polypeptides (e.g., p21, p19 and p16). For example, an antibody which recognizes the interaction between p16 and CDK4 can be used to detect or diagnose transformation of many cell types. Alternatively, agents such as antibodies which recognize CDC2, CDK2, cyclin A or cyclin D, can be used to identify the subunit composition of the cyclin complexes and thus the state of transformation of the cell.

The subject invention also relates to agents (e.g., oligonucleotides, antibodies, peptides) useful in the isolation, diagnostic or therapeutic methods described.

### Detailed Description of the Invention

Cyclins are key regulatory proteins that, in concert with cyclin-dependent protein kinases (CDKs), function to govern critical transitions and/or restriction points during the course of cell cycle progression. The present invention provides methods and reagents for developing inhibitors and/or activators of particular cell-cycle processes. As described herein, in normal eukaryotic cells, particularly human cells, various types of cyclins (such as of the A, B, and D classes) can associate with a number of different CDKs, as well as proliferating cell nuclear antigen (PCNA), and a polypeptide (p21) of an apparent molecular weight of 21kd, so as to form multiple types of complexes. For instance, the results provided below indicate that a combination of cyclins and CDKs, along with PCNA and p21, exist in at least a quaternary complex, that many combinatorial variations of the components assemble *in vivo* (e.g., different combinations of cyclin D1 or D3 with CDK2, CDK4 and CDK5), and that each of the resulting quaternary complexes may have a subtly different role in the cell cycle or in different cell types. Thus, as described below, assays can be generated to identify agents able to, for example, selectively disrupt particular complexes formed between cyclins and CDKs.

Furthermore, the present invention makes available diagnostic assays and reagents for detecting transformed cells, such as may be useful in the detection of cancer. It is demonstrated below that cellular transformation is associated with selective subunit rearrangement of the cyclin-CDK complexes. To illustrate, in cells which are transformed (either virally or by genetic aberation), the cyclin D/p21/CDK/PCNA complexes are disrupted. For instance, in virally transformed cells, CDK4 totally dissociates from cyclin D, PCNA, and p21, becoming associated instead with a 16 Kd polypeptide (termed hereinafter "p16"). Quaternary complexes containing cyclins A or B1 and p21/CDK/PCNA also undergo subunit rearrangement in transformed cells. For example, both PCNA and p21 no longer associate with CDC2-cyclin B1 binary complexes, and cyclin A complexes no longer contain p21, which is replaced instead with a 19kd protein (p19). Detecting such abnormal subunit complexes can thus be used predictively to detect of cellular transformation. For example, the present invention makes available reagents, including antibodies and nucleic acid probes, for detecting altered complex formation and/or increased levels of p16 expression in order to identify transformed cells.

Moreover, p16 is demonstrated below to exert an inhibitory effect on the activity of cyclin/CDK complexes, particularly those which include CDK4. For instance, p16 is able to inhibit the activity of cyclin D1/CDK complexes *in vivo.* As is generally known, cyclin D1 has been associated with a wide variety of proliferative diseases. Thus, the present invention identifies a potential inhibitor of cell proliferation resulting from oncogenic expression of cyclin D1.

Conversely, p16 can be used in assays to identify agents which decrease the ability of p16 to bind CDK4 and thereby relieve inhibition of cyclin/CDK4 complexes. In this embodiment, the reactivation of the CDK4/cyclin complexes disrupts or otherwise unbalances the cellular events occuring in a transformed cell. Such agents can be of use therapeutically in that activation of CDK4 complexes in cells transformed, for example, by tumor viruses, might result in enhancement of otherwise virally-suppressed cell cycle checkpoints, such as p53, and results in the accumulation of the infected cell at the checkpoint, or alternatively, in the instance of Rb phosphorylation, prematurely progesses through a checkpoint which results in cell death.

A cyclin-dependent kinase, designated CDK5, and DNA encoding CDK5 are also available as a result of the work described herein. CDK5 has been shown to co-precipitate with D-type cyclins, PCNA and p21. Thus, the present invention contemplates that CDK5 function and/or association with other members of the complex can be altered (enhanced or decreased) to effect the proliferation of a cell. If CDK5 is prevented from binding to D-type cyclin, kinase activation will be prevented. This can be effected as described below.

### I. Cyclin complexes in normal cells

The following is a description of the discovery that D-type cyclins are associated with at least three additional polypeptides (CDK, PCNA and p21) in what appears to be a quaternary complex having many possible combinatorial variations resulting in complexes that may each have distinct roles in the cell cycle, or in different cell types.

As described below and in Example 1, immunological procedures have been used to establish that D-type cyclins associate, in eukaryotic cells, with a variety of potential catalytic subunits (e.g., CDKs, such as CDK2, CDK4 and CDK5). In addition, these procedures have shown that the D-type cyclin and CDK associate with the replication factor PCNA and a polypeptide of 21 kDa apparent molecular weight.

Human cyclin D1 has been associated with a wide variety of proliferative diseases. As described herein, in human diploid cells, specifically human diploid fibroblasts, cyclin D1 is complexed with a number of other cellular proteins. Among them are the catalytic subunits CDK2, CDK4 (previously called PSK-J3), and CDK5 (also called PSSALRE). In addition, polypeptides of 21 kDa and 36 kDa are identified in association with cyclin D1. As described in Example 1, it has been shown that the 36 kDa protein is the Proliferating Cell Nuclear Antigen, PCNA. PCNA has been described as an essential accessory factor to the delta polymerase, which is required for leading-strand DNA replication and DNA repair. Cyclin D3 also associates with multiple protein kinases, p21 and PCNA, as shown herein. It is proposed that there exists a quaternary complex of D type cyclins, CDK, PCNA and p21, and that many combinatorial variations (cyclin D1, D3 with CDK2, 4 and 5) may assemble *in vivo.* These findings link a human putative G1 cyclin that is associated with oncogenesis with a well characterized DNA replication and repair factor.

### (i) Investigation of Proteins that Associate with Cyclin D

To identify proteins that specifically associate with cyclin D, anti-cyclin D1 immunoprecipitates of [³⁵S] methionine-labelled WI38 human diploid fibroblasts lysates were examined (see Example 1, Experimental Procedures). WI38 cells were initially chosen for this study because they are a relatively normal cell line that expresses reasonably high levels of cyclin D1 and a low level of cyclin D3 mRNA (Won *et al., Proc. Natl. Acad. Sci.* (1992). Human 293 transformed primary embryonal kidney cells were used as controls because they express all three D cyclin mRNAs and proteins at extremely low levels (Xiong, *et al., Cell 65*:691-699 (1991)). W138 cells express a readily detectable 35 kDa polypeptide that can be immunoprecipitated by the anti-cyclin D1 antiserum. The identity of the 35 kDa protein as cyclin D1 was confirmed by comparison of an immunoprecipitate of the same W138 cell lysate with pre-immune serum, and with a similar precipitation of 293 cell lysate with the same anti-cyclin D1 antiserum. Because of the existence of three closely related cyclin D genes in human cells, and weak cross-reactivity of the anti-cyclin D1 antibody to other cyclin D proteins, the identity of the 35 kDa band was further investigated by partial proteolytic mapping. *S. aureus* V8 partial proteolysis of the 35 kDa band revealed the same pattern as that of similar cleaved cyclin D1 synthesized *in vitro*, but not as that of cyclin D2 or D3.

In addition to the intense 35 kDa band corresponding to cyclin D1, three other major bands, p36, p33 and p21 and one minor band, p31 (where the number indicates the apparent molecular weights), appeared specifically in the anti-cyclin D1 precipitates. These polypeptides are absent from precipitates of W138 cell lysate using pre-immune serum or precipitates of 293 cell lysates with the same anti-cyclin D1 antibody. The possibility that any of these four bands, in particular p31 and p33, might be cyclin D2 or D3 was ruled out by comparing their partial V8 proteolysis patterns with those of *in vitro* translated D2 and D3. Precipitation of these polypeptides with anti-cyclin D1 serum is also not likely to be due to the presence of cross-reactive epitopes in any of these proteins, as these proteins were not detected following immunoprecipitation coupled with Western blotting using the same antibody. Experiments to identify the cyclin D1-associated proteins are described below.

### (ii) CKD5 Associates with D-Type Cyclins

It has been previously reported that in murine macrophages, cyclin D1/*cyll* associates with a polypeptide that cross-reacts with an antibody to full-length p34cdc2 of *Schizosaccharomyces pombe* (G8), but not with an antibody prepared against the C-terminus of human p34cdc2 (Draetta *et al., Cell 50*:319-325 (1987); Draetta and Beach, *Cell 54*:17-26. (1988); Matsushime *et al.,Cell 65*:701-713 (1991). Essentially identical results were presently obtained in human W138 cells, suggesting that cyclin D1 associates with a relative of human CDC2.

The G8 antibody was used to screen human cDNA expression libraries (see Example 1, Experimental Procedures), in order to isolate putative D-type cyclin-associated kinases. Thirty four G8-positive cDNA clones were identified from a HeLa cell cDNA library. Among these, 17 clones encoded CDC2 and another 14 encoded for CDK2. One of the remaining clones encodes an ORF of 292 amino acid residues (SEQ ID Nos. 1 and 2) with a predicted molecular weight of 33,283 daltons. This clone is designated CDK5, since it shares extensive amino acid identity to the known cyclin-dependent kinases (CDKs), including *S. pombe* CDC2 (53.4%), *S. cerevisiae* CDC28 (55.9%), human CDC2 (56.8%), and human CDK2 (60.3%), and associates with human D-type cyclins (see below). CDK5 encodes a sequence of DLKKYFD at amino acid sequence 86 to 92, while the corresponding region of human CDC2 has the sequence of DLK*KY*LD and CDK2 has DLK*KF*MD.

To determine whether CDK5 associates with D cyclins, an antiserum was raised against a peptide corresponding to the unique carboxy-terminal region of CDK5 (see Example 1, Experimental Procedures). This serum does not cross react with human CDC2, CDK2, or CDK4. Immunoprecipitation or Western-blotting following immunoprecipitation showed that this antiserum detected a polypeptide in cell lysate with an apparent molecular weight of 31 kDa (p31), which co-migrated with CDK5 polypeptide synthesized in vitro, and whose signal was effectively competed away by the CDK5 antigenic peptide. The identity of the 31 kDa protein precipitated by the anti-CDK5 antibody was further confirmed to be CDK5 by comparing the partial V8 proteolytic mapping of p31 with *in vitro* translated CDK5.

Immunoprecipitation of cell lysates of ³⁵S-methionine labeled W138 cells using the anti-CDK5 antiserum revealed several polypeptides, in addition to p31^{CDK5}. Among these, polypeptides of 36 kDa (p36), p35 kDa (p35), 33 kDa (p33) and 21 kDa (p21) were most prominent and specifically coprecipitated by the anti-CDK5 antiserum. All four polypeptides were absent from precipitates with the pre-immune serum or in the presence of excess amount of the CDK5 carboxy-terminal peptide.

The electrophoretic mobilities of p35 and p33 were found to be the same as that of *in vitro* translated human cyclin D1 and D3, respectively. To directly test the possibility that the CDK5-associated p35 might correspond to cyclin D1, CDK5 immunoprecipitates were blotted with anti-cyclin D1 antisera. A 35 kDa polypeptide, which co-migrated with p35^{cyclin D1}, was detected by the anti-cyclin D1 antiserum. Reciprocal blotting of anti-cyclin D1 immunocomplexes by the CDKS antiserum also revealed the presence of a 31 kDa polypeptide which had the same mobility as p31^{CDK5}. Similarly, CDK5 has also been detected in anti-cyclin D3 immunoprecipitates. These data indicate that the CDK5-associated p35 is cyclin D1, and CDKS-associated p33 is cyclin D3.

To seek conclusive evidence of the identity of the CDKS-associated p35 and p33 proteins, partial proteolytic mapping was employed (Cleveland *et al., J. Biol. Chem. 252:*1102-1106 (1977)). ³⁵S-labelled p35 purified from anti-CDK5 immunoprecipitates was subjected to partial *S. aureus* V8 protease digestion and compared with similarly treated human p35^{cyclinD1} obtained either from *in vitro* translation or from an anti-cyclin D1 immunoprecipitation. The V8 proteolytic pattern of p35 from anti-CDK5 immunoprecipitates was identical to that of cyclin D1, but distinct from that of cyclin D3. Similar experiments were also performed to confirm the identity of p33. The partial proteolytic pattern of the CDK5-associated p33 is identical to that of an vitro translated human cyclin D3, but not D1. Conversely, it has also been determined that the partial V8 digestion pattern of the cyclin D1-associated p31 is identical to CDK5 obtained either from in vitro translation or anti-CDK5 immunoprecipitation.

### (iii) CDK2 Associates with Cyclin D

The apparent molecular weight of the cyclin D1-associated p33 (e.g. in the anti-cyclin D1 precipitates), and also the cross reactivity of p33^{CDK2} with the G8 antibody suggests the possibility that p33 might be CDK2. To test this, anti-CDK2 precipitate of a [³⁵S] methionine-labelled WI38 cell lysate was compared with an anti-cyclin D1 precipitate. As expected, the anti-C terminal CDK2 serum precipitated a 33 kDA protein which was confirmed to be p33^{CDK2} by comparing the partial *S. aureus* V8 proteolysis pattern of the 33 kDa band with that of *in vitro* translated CDK2. Moreover, p33^{CDK2} comigrated with the p33 present in the anti-cyclin D1 precipitate. Reciprocally, anti-CDK2 antiserum also precipitated a 35 kDa protein which comigrated with cyclin D1.

To seek further evidence for the existence of a possible association between CDK2 and cyclin D1, a WI38 cell lysate was immunoprecipitated with anti-cyclin D1, separated on SDS-PAGE, and immunoblotted with anti-CDK2 antiserum. The anti-CDK2 antibody was raised against a carboxy-terminal peptide (Pagano *et al., EMBO J. 11*:961-971 (1992)) and its specificity was checked by immunoblotting bacterially-expressed human CDC2, CDK2, CDK3, CDK4 and CDK5. Only CDK2, and not the other four CDK proteins, was recognized by this antibody. CDK2 protein was detected in WI38 cell lystates that were immunoprecipitated with either anti-CDK2 or anti-cyclin D1, but not in lysate precipitated with pre-immune serum or with anti-CDK2 pre-incubated with competing antigenic peptides. In a reciprocal Western blot experiment, cell lysate was immunoprecipitated with anti-CDK2 and blotted with anti-cyclin D1. Cyclin D1 was detected in the anti-cyclin D1 and anti-CDK2 immunoprecipitates, but not in precipitates from either preimmune serum or anti-CDK2 antiserum pre-incubated with a competing CDK2 peptide.

To test whether CDK2 also associates with cyclin D3, immunoprecipitates formed using antiserum to the C-terminal peptide of human cyclin D3 (see Example 1, Experimental Procedures) were blotted with anti-CDK2 antiserum. CDK2 was weakly detected in the anti-cyclin D3 precipitate, but not in the control precipitate with anti-cyclin D3 antiserum pre-incubated with a competing antigen peptide.

Finally, to further confirm the association between CDK2 and cyclin D, partial proteolytic mapping experiments were conducted. Initially, attempts were made to proteolytically map the cyclin D1-associated p33 to compare it with CDK2. However, because of the comigration of CDK2 with yet another predominant protein kinase in the anti-cyclin D1 precipitates, a different proteolytic pattern was obtained. Therefore, the converse experiment was performed. The 35 kDa band in anti-CDK2 immunoprecipitates was excised from SDS-polyacrylamide gel, partially digested with V8 protease and electrophoretically separated and compared with V8 digested p35^{cyclin D1} derived either from *in vitro* translation or from an anti-cyclin D1 immunoprecipitation. The pattern of proteolytic cleavage was the same in each case.

### (iv) pSK-J3/CDK4 is the Predominant p33 Protein Associated with Cyclin D1

The difference in the proteolytic pattern of cyclin D1-associated p33 from that of CDK2 suggested that the majority of D1-associated p33 corresponds to a protein other than CDK2. A protein kinase called PSK-J3, originally identified in a screen with mixed oligonucleotide probes derived from conserved regions of serine/threonine kinases (Hanks, S.K., *Proc. Natl. Acad. Sci. USA 84*:388-392 (1987)), was believed to have cyclin D binding properties. The predicted molecular mass of PSK-J3 is 34 kDa, close to that of p33. Because of its association with D cyclins, as demonstrated below, PSK-J3 is referred to hereinafter as CDK4. *In vitro* translated CDK4, and that precipitated from a cell lysate with anti-CDK4 serum, demonstrated the same electrophoretic mobility as CDK2 and the D1-associated p33. The identify of CDK4 precipitated by the anti-CDK4 antiserum was confirmed by comparing its partial V8 mapping pattern to that of *in vitro* translated CDK4.

Immunoprecipitation-Westem blotting experiments were carried out to directly test whether the cyclin D1-associated p33 is CDK4. An anti-CDK4 serum reacted with a 33 kDa protein present in anti-cyclin D1 immunoprecipitates that has the same mobility as the CDK4 precipitated by anti-CDK4, but did not react with cell lysate precipitates of either CDK2 or CDK5. Reciprocally, the anti-CDK4 antiserum also precipitated a 35 kDa protein detected by anti-cyclin D1 antibody. To further confirm the identity of the cyclin D1-associated p33, the partial V8 digestion pattern of p33 was compared to that of immunoprecipitated CDK4 and CDK2. The cyclin D1-associated p33 displayed a very similar pattern to that of CDK4, but was quite dissimilar to that of CDK2. This result indicates that CDK4 is considerably more abundant (at least as crudely assayed by methionine labelling) than CDK2 in anti-cyclin D1 precipitates of WI38 cells. Similarly, a 33kDa polypeptide (p33) seen in anti-CDK4 immunoprecipitate has been identified to be cyclin D3 by partial V8 peptide mapping.

### (v) Association of p21 with Cyclin D1 and CDK2

In [³⁵S] methionine-labelled WI38 lysate precipitated with anti-cyclin D1 serum, a 21 kDa protein (p21) appeared to associate specifically with cyclin D1. p21 was not present in the precipitates with pre-immune serum, nor in the anti-cyclin D1 precipitate derived from 293 cells, which contain undetectable levels of cyclin D1. Specific association of p21 with cyclin D1 was further supported by the presence of a comigrating 21 kDA protein in immunoprecipitates formed with sera against CDK2, CDK4 and CDK5. When anti-CDK2 antiserum was pre-blocked with a competing CDK2 peptide, the p21 band, and also p33^{CDK2} and p35^{cyclin D1} were not seen. Similarly, p21 was also absent from anti-CDK5 immunoprecipitates if the antiserum was pre-incubated with the CDK5 carboxy-terminal antigen peptide. p21 was not recognized in Western blots by any of the anti-CDK or anti-cyclin D antibodies used in this study. Furthermore, although the total immunoprecipitable CDK2 in 293 cells is similar to that in WI38 cells, the p21 band was not present in the CDK2 immunoprecipitates from 293 cell lysates. This finding suggests that the association of CDK2 and p21 is dependent on cyclin D.

To determine whether the p21 from cyclin D1 immunoprecipitates and CDK2 immunoprecipitates correspond to the same polypeptide, the partial V8 proteolytic pattern of the p21 purified from each source were compared. They are indeed the same. The p21 precipitated by anti-CDK5 antiserum was also found to be the same as cyclin D1-associated p21. The p21 in the anti-CDK4 immunoprecipitation was also proteolytically mapped. It gave an identical pattern to the cyclin D1-associated p21. p21 does not correspond to the human max protein or p21^{ras}, as its electrophoretic mobility is faster than that of either and it was not recognized by an anti-human ras antibody on Western blots.

### (vi) Cyclin D1-Associated p36 is PCNA

As set out above, anti-cyclin D1 precipitates of WI38 cells show associated polypeptides of 21 kDa, 31 kDa and 33 kDa and also a prominent protein of 36 kDa. p36 was not detected in control precipitates, using either pre-immune serum or in 293 lysates. A 36 kDa protein, in a lower abundance was also detected in CDK2, CDK4 and CDK5 immunoprecipitates, but not in the precipitates with antiserum pre-incubated with competing peptides.

While attempting to establish the identity of the p36, four observations suggested the possibility that it might be the human proliferating nuclear antigen, PCNA. First, in an asynchronous population of proliferating WI38 cells, cyclin D1 was observed to be predominantly a nuclear protein, although the distribution is not identical to the speckled pattern of PCNA (Bravo, R. and H. MacDonald-Bravo, *EMBO J., 4*:655-661 (1985); Madsen, P. and J.E. Celis, *FEBS Lett., 193*:5-11 (1985). Second, while the level of cyclin D1 is relatively constant in mitogenically activated WI38 cells, the p36 in [³⁵S] methionine-labelled cyclin D1 immunoprecipitates was low in quiescent cells and increased at 10-14 hours after stimulation. Ten to fourteen hours after serum stimulation, many WI38 cells are in the late G1, a time which coincides with the onset of PCNA synthesis in serum-stimulated 3T3 fibroblasts (Bravo, R. and H. MacDonald-Bravo, *EMBO J., 3*:3177-3181 (1984); Celis, J.E. and A. Celis, *Proc. Natl. Acad. Sci., USA, 82*:3262-3268 (1985); Madsen P. and J.E. Celis, *FEBS Lett., 193*:5-11 (1985). Third, the apparent molecular weight of p36 is similar to that of PCNA. Finally, anti-PCNA antibody precipitated a 35 kDa polypeptide whose electrophoretic mobility is similar to that of p35^{cyclin D1}. The identify of the p36 precipitated by the anti-PCNA antibody has been confirmed as PCNA by comparing its V8 peptide map to that of *in vitro* translated PCNA.

Immunoprecipitation-Westem blot experiments were carried out to test directly the possibility that p36 is PCNA. PCNA was readily detected in anti-cyclin D1, cyclin D3, CDK2 and CDK5 immunoprecipitates, but not in the respective control precipitates. In a reciprocal experiment, cyclin D1 and CDK2 were also detected in anti-PCNA immunoprecipitates. It has not been possible to convincingly detect cyclin D3 or CDK5 in PCNA precipitates, possibly due to the low abundance of both proteins in WI38 cells and the relatively poor sensitivity of the D3 and CDK5 antisera in Western blots.

To further assess the similarity between the PCNA and the p36 polypeptide associated with cyclin D1 and CDK2, p36 bands were purified from cyclin D1 and CDK2 immunoprecipitates, separated on SDS-PAGE and their partial V8 proteolytic mapping pattern was compared with that of PCNA. Digestion of cyclin D1-associated p36 by V8 protease revealed the same pattern as that of PCNA derived from anti-PCNA immunoprecipitates and *in vitro* translated PCNA. Similarly, the digestion patterns of CDK2- and CDK5-associated p36 also match to that of PCNA. The p36 associated with cyclin D1 is PCNA. In addition, proteolytic mapping of the p21 detected in anti-PCNA immunoprecipitate demonstrated it to be the same as cyclin D1-associated p21 described above.

### (viii) Subunit complexes of other cyclins

PCNA and p21 not only associate with cyclin D-CDK to form a quaternary complex as described above, but also are present as universal components of cyclin-CDK complexes in many other normal cells example. In lysates of WI38 cells and HSF43 cells immunoprecipitated with anti-cyclin A antibody, it was found that cyclin A associates with PCNA, CDC2, CDK2, and p21.

Likewise, analysis of ³⁵S-methronine-labeled anti-cyclin B1 immunoprecipitates from WI38 and HSF43 lysates revealed a quatenary complex, comprising cyclin B1, a CDK, p21 and PCNA.

Although the experimental techniques used in this study do not formally allow a distinction between the existence of multiple pair-wise interactions between each protein, the data are most simply explained if D cyclin, PCNA, CDK and p21 form a quaternary complex. Comparison of the intensity of the methionine-labelled bands in the immunoprecipitation reactions suggest that not all the cyclin D is present in the complex, nor is all the PCNA. However, the relative intensity of the p36 (PCNA), p33 (CDK4) and p21 bands in an anti-cyclin D precipitate is very similar. The results presented herein do not rule out the possibility that cyclin D, with or without the associated proteins described here, might associate with additional partners *in vivo*. In particular, two polypeptides that migrate either side of the 97KD molecular weight marker are apparent in anti-cyclin D precipitation reaction.

PCNA has been described as an essential accessory factor to the delta polymerase, that is required both for leading-strand DNA replication and also for DNA repair (Prelich, G. *et al., Nature, 326*:517-520 (1987); Prelich G. and B. Stillman, *Cell, 53*:117-126 (1988); Toschi, L. and R. Bravo, *J. Cell Biol., 107*:1623-1628 (1988); M.K.K. Shiviji, *et al., Cell, 69*:367-374 (1992). It localizes in the nucleus at sites of active DNA synthesis and the localization of PCNA, but not its synthesis, is dependent on DNA synthesis. It was not possible to detect phosphorylation of any of the respective subunits in *in vitro* kinase reactions, suggesting that neither PCNA nor p21 is a primary substrate of cyclin D/CDK.

The cyclin D/CDK enzymes that associate with PCNA and p21 might assemble *in vivo* into a more elaborate multi-protein-DNA synthetic complex, one component of which might be the physiological substrate of cyclin D/CDK. PCNA has generally been biochemically purified from cells in a monomeric form that is unassociated with other proteins (Prelich, G. *et al., Nature 346*:760-763 (1987)). It is possible that the multi-protein complexes described in the present study were over-looked because they do not comprise the majority of the cellular PCNA. Alternatively, it is possible that PCNA has further non-DNA synthetic cell cycle regulatory roles that have not previously been described and that involve cyclin D and CDK proteins. However, the present studies do provide the first biochemical indication of a possible function of D-type cyclins, as modulators of PCNA function.

The importance of the quaternary complex is emphasized by the new discovery that cellular transformation by DNA tumor viruses is associated with selective subunit rearrangement of the cyclin D complexes, as well as other cell cycle complexes, including cyclin A, CDC2, CDK2, CDK4 and CDK5 complexes. In particular, introduction of SV40 DNA tumor virus or its oncogenic gene product large T antigen into normal human diploid fibroblasts (HDF) causes disruption of the association between cyclin D and PCNA, CDKs (such as CDK2, CDK4 and CDK5) and p21. After dissociation from cyclin D and p21, CDK4 kinase becomes associated with a 16 kDa polypeptide (p16). Similarly, SV40 transformation causes a decrease of association of p21 with cyclin A in HDF; and adenovirus-(293 cell line) or human papilloma virus- (HeLa cell line) transformed cells, p21 is completely disassociated from cyclin A. A 19 kDa peptide, p19, then appears in a complex with cyclin A. Therefore, p21 is associated with cyclin kinases only in normal, untransformed cells, whereas p16, p19 and possibly other related proteins are present in cyclin complexes in transformed cells.

### II. Cyclin Complexes in Transformed Cells

As described below, the present observations provide striking evidence that the cyclin/CDK family of enzymes that act at multiple key steps in the cell division are grossly altered in a variety of oncogenically transformed cells. In each member of the cyclin/CDK family examined, association with PCNA and p21. However, upon transformation with the DNA tumor virus SV40, or its transforming antigen (T), the cyclin D/CDK/PCNA/p21 complexes are disrupted. In transformed cells, CDK4 totally dissociates from cyclin D, PCNA, and p21 and, instead, associate exclusively with a polypeptide of 16kd (p16). Quatemary complexes containing cyclins A or B1 and p21/CDK/PCNA also undergo subunit rearrangement in transformed cells. Both PCNA and p21 are no longer associated with CDC2-cyclin B1 binary complexes. Cyclin A complexes no longer contain p21, rather, a new 19 kd polypeptide (p19) is found in association with cyclin A. This pattern if subunit rearrangement of cyclin-CDK complexes has been discovered in not only in SV40-transformed cells, but also in cells transformed with adenovirus or papilloma virus. Moreover, the pattern of subunit composition of the cyclin-CDK family was grossly abnormal in non-virally transformed cells, such as the p53-deficient cells derived from Li-Fraumeni patients, as well as the epidermal A-431 carcinoma cell, and the pharynx squamous cell carcinoma FaDu.

### (i) Disruption of cyclin D/CDK/PCNA/p21 complexes in SV40-transformed cells

While cyclin D/CDK/PCNA/p21 quaternary complexes were detected in normal fibroblast cells (e.g. WI38 ad HS68) as described above, such complexes were not detected in several other cell lines that have been virally transformed (e.g. the human 293 cell line). Given the apparent difference, the subunit compositions of cyclin D complexes in normal diploid fibroblasts and their transformed derivatives were more closely compared. The WI38 VA13, subline 2RA (hereafter referred to as VA13), is an SV40 virus-transformed derivative of the WI38 cell line. Anti-cyclin D1 immunoprecipitates of ³⁵S methionine-labeled WI38 and VA13 lysates were examined. As demonstrated above for WI38 cells, the anti-cyclin D1 antiserum precipitates a dominant 35-kD band corresponding to cyclin D1 and three major associated proteins, p36^{PCNA}, p33^{CDK4}, and p21. In SV40-transformed VA13 cells, the level of cyclin D1 is decreased by two- to threefold as determined by autoradiography of ³⁵S methionine-labeled immunoprecipitates, and by Western blotting. However, none of the three major cyclin D1-associated proteins was visibly associated with cyclin D1 in SV40 transformed VA13 cells. Reciprocal immunoprecipitations were carried out with anti-CDK4 and anti-CDK2 antisera to confirm the observed disruption of cyclin D complexes in SV40 - transformed cells. In WI38 cells, anti-CDK4 antibody precipitated CDK4 in addition to three major associated proteins, p36^{PCNA}, p35^{cyclin D1}, and p21. In SV40-transformed VA13 cells, whereas CDK4 is present at a similar level compared with untransformed WI38 cells, no detectable PCNA, cyclin D1, or p21 was present in the anti-CDK4 immunoprecipitates. Similar results were also observed in immunoprecipitates with anti-CDK2, another cyclin D-associated kinase catalytic subunit. Cyclin D1 is one of the major CDK2-associated proteins in WI38 cells, but the association is undetectable in SV40-transformed VA13 cells, even though the level of CDK2 is similar or slightly higher in the transformed cells . Also, the level of CDK2-associated p21 was reduced in transformed cells, but is present at a detectable level.

Several additional normal human diploid fibroblast cell lines and their SV40-transformed derivatives were examined. HSF43, a diploid fibroblast cell line derived from neonatal foreskin and CT10-2C-T1 (hereafter referred to as CT10), was obtained from HSP43 by introduction of a plasmid containing the SV40 large tumor antigen gene driven by a Rous sarcoma virus (RSV) promoter (Ray et al. *J Cell Biochem* 42:13-31, (1990)). The pattern of subunit composition and dissociation of the normal and transformed cells was very similar to that observed in the WI38 and VA13 cells. No cyclin D1 was seen in anti-CDK4, anti-CDK2, and anti-CDC2 immunoprecipitates prepared from T-transformed CT10 cells, but it was apparent in the HSF43 cells. Reciprocally, CDK4 was not seen in anti-cyclin D1 precipitates in CT10 cells. CDK4-associated PCNA and p21 were also not detected in CT10 cells. p21 in the T-transformed cells appeared to be replaced by a newly identified p16 protein as in the VA13 cell line. In addition, essentially identical results were also obtained from other pairs of human fibroblast cell lines, IMR-90, a normal human lung diploid fibroblast cell line and its T-transformed derivative IDH4 (Shay et al. *Biochem Biophys Acta* 1072:1-7 (1992)), as well as a pair of monkey cell lines, CV-1, a pseudodiploid kidney cell line, and its SV40-transformed derivative COS-1.

To further confirm the observed dissociation of cyclin D complexes in transformed cells, immunoprecipitation coupled with immunoblotting experiments, was performed to avoid potential artifacts that can sometimes arise in metabolic labeling experiments. Total lysates prepared from either untransformed WI38 or SV40-transformed VA13 cells were immunoprecipitated with a battery of antibodies, separated electrophoretically, and blotted with various secondary antibodies. CDK4, CDK2, and PCNA were either not detected (CDK4) or were present at dramatically decreased levels (CDK2 and PCNA) in anti-cyclin D1 precipitates derived from SV40-transformed cells. In each case, direct immunoblotting confirmed that the absolute level of CDK4, CDK2, and PCNA is similar in normal and transformed cells. Reciprocally, cyclin D1 was not detected in either anti-CDK2 or anti-CDK4 precipitates derived from transformed VA13 cells. The same results were also obtained from each of the other pair of cell lines, HSF43 and CT10.

### (ii) Subunit rearrangement of cyclin A and B complexes in transformed cells

The subunit composition of cyclin A complexes in untransformed WI38 cells and SV40-transformed VA13 cells were examined. As described above, cyclin A associates with p36^{PCNA}, p33^{CDC2} p33^{CDK2}, and p21 in WI38 cells. In SV40-transformed VA13 cells, the level of both cyclin A itself and cyclin A-associated CDC2/CDK2 increased approximately threefold. Reciprocally, the level of CDC2- and CDK2-associated cyclin A is also increased two- to threefold..

The level of cyclin A-associated p21, as determined by immunoprecipitation of ³⁵S methionine-labeled cell lysates, is reduced in VA13 cells as compared with WI38 cells. A low level of p21 is also present in the immunoprecipitates prepared from VA13 cells using antisera against CDK2, the major catalytic partner of cyclin A. Consistently, the same results were obtained using HSF43 and CT10 cells, in addition to normal IMR-90 cells versus their T-transformed IDH cells, and monkey CV-1/COS-1 pair cell lines.

A number of other polypeptides were noticed in cyclin A precipitates that are specific to untransformed cells or to transformed cells. For example, a protein with a molecular mass of 42 kD is seen as a predominant band in untransformed WI38 or HSF43 cells but not in transformed VA13 or CT10 cells. Conversely, a polypeptide of 95 kD, which might correspond to the T antigen, is seen only in transformed cells, as is a 19 kd polypeptide (discussed below).

The subunit composition of cyclin B1 complexes in normal and transformed cells was also examined. In common with cyclin D1, the quaternary complex of cyclin B1/CDK/p21/PCNA is altered in both SV40-transformed VA13 cells and T-transformed CT10 cells as compared with nontransformed WI38 and HSF43 cells. Thus, neither PCNA nor p21 associates with cyclin B/CDK in the transformed cells. However, as anticipated, CDC2 is associated with cyclin B1 (p62) in VA13, CT10, 293, and HeLa cells.

### (iii) Cyclin and CDK complexes in other DNA tumor virus-transformed cells

To investigate whether cyclin-CDK complexes are also altered in other human tumor cells, papilloma virus-containing cervix carcinoma HeLa cells and adenovirus-transformed primary kidney 293 cells were examined for the subunit composition of various cyclin and CDK complexes. Both of these cell lines have been used widely in biochemical studies of the mammalian cell cycle. Cyclin D1 is expressed at a low level in HeLa cells and is barely detectable in 293 cells. The subunit composition of other cyclin/CDK complexes, however, can be investigated. The well-established association of CDC2-cyclin B1, CDC2-cyclin A, and CDK2-cyclin A were all readily detected in both HeLa and 293 cells. No p21 was seen, however, in anti-CDC2, anti-CDK2, anti-cyclin A, or anti-cyclin B1 immunoprecipitates from either HeLa or 293 cells. Thus, the quaternary complexes of cyclin A/CDK/p21/PCNA and cyclin B1/CDK/p21/PCNA are either not assembled or are present at extremely low levels in these DNA tumor virus-transformed cells. In fact, a new cyclin A complex appeared, in which p21 seems to be replaced by a polypeptide of 19 kD. A similar 19 kD polypeptide is present in anti-cyclin A precipitates from SV40-transformed human VA13, CT10, IDH4, and even monkey COS-1 cells. Furthermore, in 293 and HeLa cells, the subunit composition of CDK4 is identical to that of the T-antigen-transformed cells. Cyclin D1 is not associated with CDK4 (trivially attributable to absence of cyclin D from the cell), but PCNA and p21 are also absent. p21 has been replaced by p16 in HeLa and 293 cells. Interestingly, CDK4-associated p16 is detected at very low levels in untransformed WI38 or HSF43 cells. Proteolytic cleavage mapping in each case revealed that the CDK4-associated p16 from HeLa, 293, VA13 and WI38 cells is identical, but different from p21. The presence of p19 and p16 in cells transformed by three different viruses and p16 in untransformed cells indicates that they are not virally encoded proteins.

### (iv) Alteration of cyclin and CDK complexes in Li-Fraumeni cells

Fibroblasts from familial Li-Fraumeni patients exhibit an elevated rate of spontaneous abnormalities, including aneuploidy and immortalization (Li et al. *J Natl Cancer Inst* 43:1365-1373 (1969); and Bischoff et al. *Cancer Res* 50:7979-7984 (1990)). These cells do not contain any known viruses; instead single-base heterozygous mutations of the tumor suppressor gene p53 are the only reported germ-line alteration. Escape from senescence and spontaneous immortalization of Li-Fraumeni fibroblasts during continued passage *in vitro* is associated with secondary loss of the wild-type p53 allele. ³⁵S methionine-labeled cell lysates from a spontaneously immortalized Li-Fraumeni cell line, LCS041 (passage >170) were immunoprecipitated with a variety of antibodies and analyzed by SDS-PAGE. The level of most of these proteins in LCS041 cells is similar to that in normal diploid fibroblasts as determined by metabolic labeling and immunoblotting, except that cyclin A is expressed at a much lower level compared with normal fibroblasts. The subunit composition of cyclin and CDK complexes is grossly abnormal in LCS041 cells. cyclin B1-CDC2 complexes exist, as in HeLa and 293 cells, and are not associated with p21 or PCNA. The cyclin D1-CDK4 association is reduced to a barely detectable level in LCS041 cells, and both PCNA and p21 are absent. p21 was not detected in any of the cyclin-CDK complexes investigated in LCS041 cells and did not appear to be replaced by p16 or p19 proteins as occurs in T-antigen-transformed fibroblasts. The level of PCNA associated with cyclin-CDK complexes is also dramatically reduced in every case. Direct immunoblotting demonstrated that the abundance of PCNA is higher in LCS041 cells, compared with normal fibroblasts, but PCNA is dramatically reduced or entirely absent from immunocomplexes isolated with anti-CDC2, CDK2, CDK4, cyclin A, cyclin B1, and cyclin D1. Essentially identical results were also obtained by use of another Li-Fraumeni cell line, LCS087.

### III. Cloning of p16, and inhibitor of CDK4

The two-hybrid screening system (Fields et al. *Nature* 340:254 (1989)) was utilized to search for proteins that could interact with human CDK4, and more specifically, to isolate a cDNA encoding p16. Two-hybrid screening relies on reconstituting a functional GAL4 activator from two separated fusion proteins: the GAL4 DNA-binding domain fused to CDK4, GAL4db-CDK4; and the GAL4 activation domain fused to the proteins encoded by HeLa cDNAs, GAL4ad-cDNA. YPB2 was used as the recipient yeast as it is a strain that contains two chromosomal genes under the control of two different GAL4-dependent promoters: HIS3 and LacZ. YPB2 was transformed with a mixture of two plasmids encoding, respectively, the GAL4db-CDK4 and the GAL4ad-cDNA fusions; several clones were obtained that grew in the absence of histidine and that turned blue in the presence of β-gal. From DNA sequencing data it was determined that each of the positive clones derived from the same gene, although one group represented mRNAs with a shorter 3' end. The sequence of these cDNAs contained, in-phase with the GAL4ad, an open reading frame encoding a protein of 148 amino acids with a predicted molecular weight of 15,845 daltons (see SEQ ID Nos. 3 and 4). This protein is referred to hereinafter as INK4 (inhibitor of CDK4; see below). The sequence of p16INK4 was compared by standard methods with those present in the currently available data banks and no significant homologies were found.

To test if p16INK4 would specifically bind CDK4, YPB2 were cotransformed with the GAL4ad-p16INK4 fusion as well as with several target GAL4db fusion constructs containing, respectively, cdc2, CDK2, CDK4, CDK5, PCNA and Snfl (a fission yeast kinase). Transformed cells were plated with and without histidine. Only the GAL4db-CDK4 fusion interacted with GAL4ad-p16INK4 to an extent which allowed growth in the absence of histidine, indicating that this pair of fusion proteins specifically reconstituted a functional GAL4 activator able to enhance the expression of the HIS3 gene. The same result was obtained when the ability to transactivate the expression of the β-galactosidase gene was assayed.

The specificity of this interaction was further demonstrated in a cell-free system, by mixing *in vitro* translated (³⁵S)-labeled CDKs with a purified bacterially-produced fusion protein consisting of glutathione-S transferase (GST) linked to p16INK4 (17). The GST-p16INK4 fusion was recovered by binding to glutathione-sepharose beads and the association of each CDK was analyzed by gel electrophoresis. Consistent with the previous observations, GST-p16INK4 bound much more efficiently to CDK4 than to cdc2, CDK2 or CDK5.

Since the predicted molecular weight of p16INK4 is close to 16 Kd, the identity of p16INK4 as the CDK4-associated p16 protein found in transformed cell lines (see above) was determined. Two *in vitro* translation products of 15 KD and 17 KD were obtained from the p16INK4 cDNA. These products, as well as the CDK4-associated p16 protein from HeLa cells were treated with N-chlorosuccinimide. The partial NCS-proteolytic pattern of the 17 KD cDNAINK4-derived product was very similar to the pattern obtained with the CDK4-associated p16 protein from HeLa cells, strongly suggesting that the p16INK4 cDNA actually corresponds to p16. Partial digestion with V8 protease of the 17 KD cDNAINK4-derived product and p16 also yielded similar patterns. It is interesting to note that the p16INK4 protein overexpressed in insect cells has an electrophoretic mobility of 15 KD, and its NCS proteolytic map is identical to that obtained with the 15 KD cDNA derived product. This suggests that the actual p16INK4 found in human cells and the 17 KD *in vitro* translation product correspond to posttranslationally modified proteins. The fact that the p16INK4 protein overexpressed in insect cells interacts with CDK4 suggests that this modification is not essential for the interaction (see below).

The identity between p16INK4 and the CDK4-associated protein p16 was further confirmed using antibodies raised against the purified GST-p16INK4 fusion protein. Several human cell lines were used for this experiment: a normal cell line WI38, derived from normal lung fibroblasts; the VA13 cell line derived from WI38 by transformation with the SV40 T-antigen; and HeLa cells. As set out above, anti-CDK4 immunoprecipitates of WI38 revealed the association of CDK4 with cyclin D1, PCNA, p21 and p16. In contrast, in VA13 and HeLa cells CDK4 is only associated with p16. Anti-p16INK4 immunoprecipitates contained a protein with an apparent molecular weight of 16 KD which was readily detectable in the two transformed cell lines, VA13 and HeLa but to a lesser extent in the normal cell line WI38. This protein not only had the same electrophoretic mobility as the p16 protein coimmunoprecipitated with anti-CDK4 serum, but also had an identical NCS partial proteolytic pattern. In addition to p16INK4 a protein of 33Kd was observed in anti-p16 coimmunoprecipitates that was shown to be identical to CDK4 by V8-proteolytic mapping.

Northern analysis of the transcripts present in WI38 and VA13 cells indicated that the p16INK4 mRNA was around many times less abundant in WI38 cells compared to VA13 cells. This difference approximately corresponded to the observed difference in the amount of p16 protein between the two cell lines, suggesting the possibility that p16INK4 expression might be regulated at a transcriptional or post-transcriptional level. Indeed, in three non-virally transformed cell lines the expression of p16INK4 could not be detected even after overexposure of the gel.

To study the biochemical consequences of the interaction of p16INK4 with CDK4, active CDK4-cyclin D complexes have been reconstituted *in vitro* by standard protocols (Kato et al. *Genes Der* 7:331 (1993); and Ewen et al. *Cell* 73:487 (1993)). The three relevant components, CDK4, p16INK4 and cyclin D1, were expressed in baculovirus-infected insect cells. Extracts were prepared from metabolically (³⁵S)-labeled insect cells that separately overexpressed p16INK4, CDK4 or cyclin D1, as well as from cells overexpressing both CDK4 and cyclin D1. In response to increasing amounts of p16INK4, corresponding decreases in the ability of CDK4 to phosphorylate Rb was observed. This inhibition correlated with the association between p16INK4 and CDK4 as detected by immunoprecipitation. No inhibition was observed when CDK2-cyclin D2 complexes were used in a similar assay. To confirm that the inhibition of CDK4 was due to p16INK4, a His-tagged p16INK4 fusion protein (His-p16INK4) was created to have an amino terminal extension of 20 amino acids containing a tract of 6 histidine residues. This fusion protein was overexpressed in baculovirus-infected insect cells, and was purified by virtue of the high-affinity association of the histidine tract to nickel-agarose beads. The His-p16INK4 protein preparation was shown to be >90% pure, and inhibited the activity of the CDK4-cyclin D1 complex under conditions similar to those used for inhibition by the whole lysates.

### IV. Uses of the Invention

Based on work described herein, it is possible to detect altered complexes of cyclins CDK, PCNA, p21, p19, and p16 in cells obtained from a tissue or biological sample, such as blood, urine, feces, mucous, saliva, or biopsis (including cytological preperations). This has potential for use for diagnostic and prognostic purposes since, for example, there is an apparent link between alteration of a subunit composition of composition of complexes and cellular transformation or abnormal cell proliferation. Diagnostic and therapeutic methods described herein can be used to assess an individual's disease state or probability of developing a condition associated with or otherwise indicated by such rearrangements of complex subunits, and to monitor therapy effectiveness (by assessing the effect of a drug or drugs on subunit rearrangement).

For example, an agent can be developed that recognizes the interactions between CDKs, cyclins, PCNA and low molecular weight polypeptides (such as p21, p19 and p16). The agent can then be contacted with the sample of cells for which the transformation state is to be tested; presence of particular subunits in a complex will be indicative of transformation. For example, a CDK4-p16 complex will be indicative of transformation, as will a cyclin A-p19 complex. Alternatively, agents which recognize different subunits can be used in conjunction, to determine the presence of interactions among the subunits. For example, an agent which recognizes p21 can be used in conjunction with an agent which recognizes a cyclin or a cyclin kinase, to determine whether p21 is complexed with either the cyclin or the cyclin kinase.

Antibodies specifically reactive with compounds of the quaternary complexes can be produced, using known methods, to be used as agents in these methods. For example, antisera can be produced by injecting an appropriate host (e.g., rabbits, mice, rats, pigs) with the D-type cyclin against which anti sera is desired and withdrawing blood from the host animal after sufficient time for antibodies to have been formed. Monoclonal antibodies can also be produced using known techniques. Sambrook, J. *et al., Molecular Cloning: A Laboratory Manual*, Cold Spring Harbor Laboratory, Cold Spring Harbor, NY (1989); Hallow, E. and D. Lane, *Antibodies: A Laboratory Manual*, Cold Spring Harbor Press, New York (1988). Antibodies specifically reactive with CDK5, CDK4 and other CDKs in general, p21, p19, p16, and cyclins can also be produced using known methods.

In an illustrative embodiment, antibodies are raised against the CDK4/p16 complex. To facilitate the production of such complexes in immunogenic form, the CDK4/p16 complex can be generated by chemical cross-linking. In another embodiment, a CDK4/p16 fusion protein is generated using single chain antibody technology to introduce an unstructured polypeptide linker region between the polypeptide sequences derived from each of the proteins. This linker can facilitate enhanced flexibility of the fusion protein allowing each subunit to freely interact with the other, reduce steric hindrance between the two fragments, as well as allow appropriate folding of each fragment to occur. The linker can be of natural origin, such as a sequence determined to exist in random coil between two domains of a protein. Alternatively, the linker can be of synthetic origin. For instance, the sequence (Gly₄Ser)₃ can be used as a synthetic unstructured linker. Linkers of this type are described in Huston et al. (1988) PNAS 85:4879; and U.S. Patent No. 5,091,513. Thus, immunogenic complexes resembling the CDK4/p16 complex can be generated, and subsequent antibodies isolated which recognize the complex but not the isolated subunits.

In yet another embodiment, the present invention particularly contemplates assays and kits for detecting p16 levels in cells. Antibodies specific for p16 (as described herein), or nucleic acid probes directed to detecting mRNA levels of R¹⁶ transcripts, can be used to detect transformed cells. As described above, the level of p16 mRNA, and presumably p16 protein, is elevated in transformed cells relative to normal cells. Thus, detecting the level of p16 gene expression is diagnostically useful in determining the presence of transformed cells. In an illustrative embodiment, *in situ* hybridization assays can be performed using nucleic acid probes directed to p16 sequences and generated by standard protocols (see, for example, the Taub et al. U.S. Patent No. 4,820,630; Falkow et al. U.S. Patent No. 4,358,935; and the Bresser et al. U.S. Patent No. 5,225,326).

A preferred assay of the present invention allows quantitative differences to be ascertained in p16 levels between transformed which a characterized by p16/CDK4 complexes, and untransformed cells. Briefly, cells, either as single cell suspensions or as tissue slices may be deposited on solid supports such as glass slides. Alternatively, cells are placed into a single cell suspension of about 10⁵ -10⁶ cells per ml. The cells are fixed by choosing a fixative which provides the best spatial resolution of the cells and the optimal hybridization efficiency when nucleic acid probes are employed, or the optimal binding affinity and specificity when anti-p16 Abs are utilized.

In the instance of nucleic acid probes, the hybridization can then carried out in the same solution which effects fixation. This solution contains both a fixative and a chaotropic agent such as formamide. Also included in this solution is a hybrid stabilizing agent such as concentrated lithium chloride or ammonium acetate solution, a buffer, low molecular weight DNA and/or ribosomal RNA (sized to 50 bases) to diminish non-specific binding, and a pore forming agent to facilitate probe entry into the cells. Nuclease inhibitors such as vanadyl ribonucleoside complexes may also be included.

To the hybridization solution is added the p16 probe, to hybridize with a target polynucleotide. The most preferable probe is a single-stranded anti-sense probe. For hybridization to cellular mRNA, a probe of approximately 75 to 150 bases in length is used. The hybridization solution containing the probe is added in an amount sufficient to cover the cells when using immobilized cells. The cells are then incubated at a optimal temperature.

The probes may be detectably labeled prior to the hybridization reaction. Alternatively, a detectable label may be selected which binds to the hybridization product. Probes may be labeled with any detectable group for use in practicing the invention. Such detectable group can be any material having a detectable physical or chemical property. Such detectable labels have been well-developed in the field of immunoassays and in general most any label useful in such methods can be applied to the present invention. Particularly useful are enzymatically active groups, such as enzymes (see *Clin. Chem.*, 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescent markers (see Clin. Chem., 25:353 (1979); chromophores; luminescent compounds such as chemiluminescent and bioluminescent markers (see *Clin. Chem.,* 25:512 (1979)); specifically bindable ligands; proximal interacting pairs; and radioisotopes such as ³H, ³⁵ S, ³² P, ¹²⁵I and ¹⁴C.

In similar fashion, anti-p16 antibodies can be labeled and used to detect the presence of p16 protein in samples of cells.

The present invention also includes a method of screening compounds or molecules for their ability to inhibit or suppress the transformation of a cell. A compound or molecule to be assessed for its ability to inhibit transformation is contacted with the cells, under conditions appropriate for entry of the compound or molecule into the cells. Transformation of the cell will result in selective rearrangement of subunits in the cyclin complexes. Comparison of the rate or extent of rearrangement in the presence of the compound or molecule being assessed with that of an appropriate control (e.g., the same type of cells without added test drug) will demonstrate the ability or inability of the compound or molecule to inhibit subunit rearrangement. Drugs which inhibit subunit rearrangement are also the subject of this invention.

For example, protein kinase-D type cyclin complex formation can be prevented in a direct manner by, for example, introducing into cells a drug or other agent which binds the protein kinase or the D-type cyclin or otherwise interferes with the physical association between the cyclin and the protein kinase it activates (e.g., by intercalation) or disrupts the catalytic activity of the enzyme. This can be effected by means of antibodies which bind the kinase or the cyclin or a peptide or low molecular weight organic compound which, like the endogenous D-type cyclin, binds the protein kinase, but whose binding does not result in activation of the enzyme or results in its being disabled or degraded. Peptides and small organic compounds to be used for this purpose can be designed, based on analysis of the amino acid sequences of D-type cyclins, to include residues necessary for binding and to exclude residues whose presence results in activation. This can be done, for example, by systematically mapping the binding site(s) and designing molecules which recognize or otherwise associate with the site(s) necessary for activation, but do not cause activation. As described herein, there is differential expression in tissues of D-type cyclins, as well as the complexes formed with CDKs. Thus, it is possible to selectively decrease mitotic capability of cells by the use of an agent (e.g., an antibody or anti-sense or other nucleic acid molecule) which is designed to interfere with (inhibit) the activity and/or level of a complex comprising a particular D type cyclin and CDK. For example, in treating tumors involving the central nervous system or other non-hematopoietic tissues, agents which selectively inhibit cyclin D1 might be expected to be particularly useful, since D1 has been shown to be differentially expressed (expressed at particularly high levels in cells of neural origin).

Formation of complexes of D-type cyclin, CDK, PCNA and p21 can also be prevented in a similar manner as that described above for inhibiting CDK/D-type cyclin complex formation. That is, complex formation can be prevented directly (e.g., by means of a drug or agent which binds a component of the complex or otherwise interferes with the physical association of complex components. Complex formation can also be prevented in an indirect manner, such as by preventing transcription and/or translation of DNA and/or RNA encoding a component of the complex, in a similar manner to that described above for blocking D-type cyclin-protein kinase complex formation. Alternatively, complex formation can be prevented indirectly by degrading one or more of its constituents.

For example, a drug which selectively inhibits the ability of cyclin D2 or cyclin D3 to form a quaternary complex can be used. Each of the other complex constituents is also a target whose function or availability for complex formation can be altered. For example, CDK2, CDK4, CDK5 and other cyclin dependent kinases which complex with a D-type cyclin can be inhibited or enhanced, either in terms of their function or their availability for incorporation into the quaternary complex. Drugs or agents which alter PCNA function or availability, or which alter p21 function or availability, or p16 function or availability can also be used to inhibit or enhance cell division. In the case of each quaternary complex constituent, it is possible to introduce into cells an agent, such as a small peptide or other organic molecule, which mimics the complex constituent in terms of binding but lacks its active region(s), which results in formation of complexes lacking the activity or interactions of the normally-produced complex.

Direct inhibition of complex formation can also be nonspecific (i.e., can affect the majority of cells or all cells in which the D-type cyclin-containing quaternary complex is formed). This can be done, for example, by introducing into cells a drug which inhibits function or availability of a common component of the quaternary complex (e.g., PCNA) or by introducing a mixture or cocktail of drugs, which together inhibit all D-type cyclins.

Alternatively, indirect inhibition of quaternary complex is possible. That is, a drug or agent which acts to cause less of a complex constituent (e.g., D-type cyclin, CDK, PCNA or p21) available can be used. Such drugs or agents include those, such as anti-sense oligonucleotides, which block transcription or translation and those, such as an enzyme, which degrade complex constituents, either prior to or after their incorporation into a quaternary complex.

Drugs or agents useful in the present method of altering, particularly inhibiting, cell cycle start and, thus, cell division, can be existing compounds or molecules (e.g., small organic molecules, anti-sense oligonucleotides, and inorganic substances) or materials designed for use in the present method. In either case, such drugs can be identified by the method of the present invention.

In a particular embodiment of the present invention, interaction trap assays can be used to identify agents able to disrupt complex formations with cyclins, particularly D-type cyclins, as well as p16/CDK4 complexes. For instance, two hybrid assays described above can be utilized in such assays. In an illustrative embodiment, GAL4 activator fusion construct as described herein containing CDK4 and p16 are used to measure the ability of a candidate agent to disrupt the formation of CDK4/p16 complexes. Cells transfected with each of the constructs are contacted with a candidate agent, and the level of expression of a reporter gene (such as β-galactosidase, as above) is detected. A decrease in expression can be indicative of an inhibitor of the complex.

Furthermore, a three hybrid assay can be preformed to detect inhibitors of CDK4/p16 which do not inhibit CDK/cyclin complexes. For example, constructs can be generated in which p16 and a D-type cyclin each are part of fusion proteins which constitute the DNA binding domains of discrete proteins recognizing different DNA sequences. CDK4 is then generated as fusion protein with an activating domain, such its interaction with the p16 fusion protein results in expression of one reporter gene, while its interaction with the cyclin D fusion protein drives expression of different reporter gene. For instance, the p16 fusion protein can drive expression of a luciferase gene, while the cyclin D construst provides expression of a drug-resistance marker. Thus, in the presence of an agent which inhibits p16/CDK4 interactions, the expression of the drug resistance marker indicates that cyclin D/CDK4 complexes are not disrupted.

Moreover, given the inhibitory ability of p16, the present invention further contemplates the use of such knowledge to generate peptidomimetric analogs of particular portions of p16, which can be used as anti-proliferative agents. The CDK4 binding interactions with p16 can be easily ascertained, such as by use of mutagenesis and the above described interaction trap assay. Likewise, peptide fragments derived from p16 can be assayed for their ability to disrupt CDK4/cyclin complexes as described above.

Once an appropriate drug or agent has been identified, it can be administered to an individual, particularly a human or other vertebrate, by any route effective in introducing the drug or agent into cells in sufficient quantity to have the desired effect (i.e., alteration of cell division). For example, a selected drug can be administered intravenously, intramuscularly, by direct injection into a tumor, via the gastrointestinal tract (e.g., orally), intraperitoneally or intranasally. In some cases, ex vivo administration is appropriate (e.g., in instances where blood or bone marrow is removed from the body, treated and returned to the body).

Generally, the drug or agent used to alter cell division will be included in a formation which can also include a physiological carrier (e.g., a buffer or physiological saline), stabilizers, an adjuvant, and flavoring agents. The quantity of the drug to be administered can be determined empirically and will vary depending on considerations such as the age, weight and height of the recipient and the severity of the condition to be treated.

Antibodies specifically reactive with D-type cyclins of the present invention can also be produced, using known methods. For example, anti-D type cyclin antisera can be produced by injecting an appropriate host (e.g., rabbits, mice, rats, pigs) with the D-type cyclin against which anti sera is desired and withdrawing blood from the host animal after sufficient time for antibodies to have been formed. Monoclonal antibodies can also be produced using known techniques. Sambrook, J. et al., Molecular Cloning: A Laboratory Manual, Cold Spring Harbor Laboratory, Cold Spring Harbor, NY (1989); Hallow, E. and D. Lane, Antibodies: A Laboratory Manual, Cold Spring Harbor Press, New York (1988). Antibodies specifically reactive with CDK5, can also be produced using known methods.

The present invention also includes a method of screening compounds or molecules for their ability to inhibit or suppress the function of a cyclin, particularly a D-type cyclin. For example, mutant cells as described herein, in which a D-type cyclin such as D1 or D3, is expressed, can be used. A compound or molecule to be assessed for its ability to inhibit a D-type cyclin is contacted with the cells, under conditions appropriate for entry of the compound or molecule into the cells. Inhibition of the cyclin will result in arrest of the cells or a reduced rate of cell division. Comparison of the rate or extent of cell division in the presence of the compound or molecule being assessed with cell division of an appropriate control (e.g., the same type of cells without added test drug) will demonstrate the ability or inability of the compound or molecule to inhibit the cyclin. Existing compounds or molecules (e.g., those present in a fermentation broth or a chemical "library") or those developed to inhibit the cyclin activation of its protein kinase can be screened for their effectiveness using this method. Drugs which inhibit D-type cyclin are also the subject of this invention.

The present invention also includes a method of screening compounds or molecules for their ability to alter formation of the quaternary complex described herein. This method is carried out in much the same way as the method, described above, for identifying compounds or molecules which inhibit a D-type cyclin. In the subject method, the compound or molecule to be tested and cells in which D-type cyclin-containing complex is formed are combined, under conditions appropriate for complex formation to occur and entry into cells of the compound or molecule being tested. Complex formation can be determined, as described herein. Inhibition of a complex constituent or of complex formation will result in arrest of the cells or a reduced rate of cell division. Comparison of the rate or extent of cell division in the presence of the compound or molecule being tested with the rate or extent in the absence of the compound or molecule will demonstrate whether it has an effect on cell division (i.e., division to a lesser extent in the presence of the compound or molecule tested than in its absence is an indication the compound or molecule is an inhibitor). Drugs or agents which inhibit complex formation and, as a result, cell division, are also the subject of this invention.

The present invention will now be illustrated by the following examples, which are not intended to be limiting in any way.

### EXAMPLE 1

### Demonstration that D-Type Cyclins Associated with Multiple Protein Kinases and the DNA Replication and Repair Factor PCNA

### (i) Experimental Procedures

### Cells

Human diploid lung fibroblast WI38 cells were obtained from American Type Culture Collection at passage 13 and were grown in Dulbecco-Modified Eagle media supplemented with 10% fetal bovine serum and used between passages 16-22. 293 cells were cultured similarly.

### Antibodies

To raise anti-cyclin D1 antibody, a 609 bp DNA restriction fragment encoding 202 amino acid residues (∼25 kDa) of human cyclin D1 amino-terminal region (the NCol fragment from nucleotides 143 to 751 in Figure 2 of Xiong, *et al., Cell 65*:691-699 (1991 and *Current Biology 1*:362-364 (1991)) was subcloned into a phage T7 expression vector, pET-3d (Studier, *et al., Methods in Enzymology, 185*:60-89 (1990)) and introduced into *E. coli* strain BL21 (DE3). Bacterial extracts were prepared in lysis buffer (150 mM NaCl, 50 mM Tris-HCl, pH7.5 and 10% glycerol) by disrupting cells with sonication and clarifying the supernatant by centrifugation at 20,000 g for 10 minutes. Pellets containing insoluble cyclin D protein was resuspended in lysis buffer supplemented with 8 M urea, after 30 minutes shaking at room temperature, the suspension was centrifuged again at 20,000 g for 10 minutes. Pellets containing insoluble cyclin D protein was resuspended in SDS sample buffer and separated on 10% SDS-polyacrylamide gel. The 25 kDa cyclin D protein was visualized and excised after staining the gel with 0.25M KC1 in the cold room. Gel slices were further crushed by repeated passage through an 18 gauge needle and cyclin D protein was extracted by incubating the crushed gel particles with PBS containing 0.1% SDS at 42°C for several hours and used for injection of rabbits. To affinity purify the anti-cyclin D1 immunoglobulins, bacterially produced p25 proteins were cross-linked to the Reacti-Gel (6X) according to the manufacturer's instruction. The affinity column was washed with excess volume of PBS containing 0.05% Tween-20 before and after crude serum was applied to the column. Bound immunoglobulins were eluted with Glycine-NaCl (pH2.5) into 1.5 M Tris-HCl, pH8.5 to instantly neutralize the antibodies. To reduce the high background caused by immunoglobulin proteins, affinity purified anti-cyclin D1 was crosslinked to protein A agarose beads according to Harlow and Lane, *Antibodies: a laboratory manual,* Cold Spring Harbor Laboratory Press, NY (1988). On Western blots, the anti-cyclin D1 antiserum weakly cross-reacts with bacterially produced human cyclin D2, very poorly with bacterially produced human cyclin D3, and detects a single band from total WI38 cell lysates. In the immunoprecipitations with RIPA buffer (0.1% SDS), more than 90% of cyclin D1-associated p36, p33, p31 and p21 are disappeared while the amount of cyclin D1 remained to be the same as that in the immunoprecipitations with NP40 (0.5%) buffers.

For anti-CDK5 antibody production a peptide CYFSDFCPP, with the underlined amino acid residues corresponding to the carboxy-terminal region of CDK5, was synthesized. The peptide was coupled to keyhole limpet hemocyanin (Pierce) which was then used to immunize rabbits by standard protocols (Green, *et al., Cell 28*:477-487 (1982)).

Anti-cyclin D3 peptide antibody was similarly raised against a synthetic peptide CDELDOASTPTDVRPIDL, with the underlined region corresponding to the carboxy-terminal region of human cyclin D3. The rabbit was later stimulated with bacterial produced full length human cyclin D3. Cyclin D3 specific immunoglobulins were purified on an affinity column in which the 17-mer cyclin D3 peptides were crosslinked to the Reacti-Gel (6X). The affinity purified anti-cyclin D3 peptide antibody does not cross-react with bacterially produced cyclin D1 or D2 on Western blots and does not immunoprecipitate cyclin D1 from W138 cell lysates.

The antiserum against S. pombe p34^{cdc2} (G8) was described before (Draetta, *et al., Cell 50*:319-325 (1987)). Human auto-immune anti-PCNA antiserum are generally known. Affinity purified anti-PCNA monoclonal antibody used in Western-blots was purchased from Boehringer Mannheim. Affinity purified anti-PCNA monoclonal antibody used in immunoprecipitation was purchased from Oncogene Science. Anti-CDK2 peptide antiserum was previously described (Pagno, *et al., EMBO J* 11:961-971 (1992)) and does not cross-react with CDC2, CDK4 and CDK5 polypeptides. Anti-CDK4 antiserum was raised against a fusion protein of glutathione S transferase (GST) and a C-terminal portion of CDK4. It does not cross-react with CDK2 and CDK5.

### (ii) Screening Human cDNA Expression Library

A human HeLa cell cDNA expression library constructed in lambda ZAP II (#936201) was from Stratagene. Human p34^{cdc2} was highly insoluble when produced from bacteria. The conventional antibody screening method (Young and Davis, *Proc. Natl. Acad. Sci. 80*:1194-1198 (1983)) is suitable only when there is sufficient amount of soluble recombinant proteins in phage plaques. The screening method, therefore, was modified to include a step which involved the use of 6M guanidine to solubilize recombinant proteins after they have been transferred to nitrocellulose paper, a procedure which was initially developed to produce refolded recombinant proteins with certain activities (Vinson, *et al., Gene Dev. 2*:801-806 (1988)). Two million phage plaques from the λZAP II HeLa cDNA library were screened with antiserum against S. pombe p34^{cdc2} (G8). After overlaying phage plaques with IPTG-impregnated nitrocellulose filters for 4 hours at 42°C, the filters were removed from culture dishes and were then treated with 6 M guanidine-HCI in a buffer containing 25 mM Hepes, pH7.0, 50 mM NaCl, 2 mM DTT for 10 min at 25°C. The filters were washed free of guanidine with Tris-buffered saline before antibody incubation. This procedure enhanced our antibody detection signal greatly which probably was due to the solubilization of bacterial-produced polypeptide precipitates by guanidine. The G8-positive cDNA clones subcloned into pBluescript SK vector (Stratagene) and sequenced from both directions using ABI automated DNA sequencer (Model 373A). For sequence homology search, the FASTA program was used (Pearson and Lipman, *Proc. Natl, Acad. Sci. 85*:2444-2448 (1988)).

### (iii) Immunoprecipitation and Western-Blotting

For metabolic labelling with [³⁵S] methionine, sub-confluent (40-60%) cells were washed twice with prewarmed labelling media (methionine-, cystine-free DMEM [ICN] supplemented with 10% dialyzed fetal bovine serum, [GIBCO]). After 30 minutes incubation with the labelling media, [³⁵S] methionine (Trans³⁵S-label, ICN) was added to media (approximately 200 µCi/ml) and continued to incubate for four to six hours before lysis. All steps of immunoprecipitations were carried out in the cold room. Cells from 40 to 60% confluent 150 mM dish were washed twice with cold PBS and scraped into NP-40 lysis buffer (50 mM Tris-HCl, pH7.4, 150 mM NaCl, 20 mM EDTA, 0.5% NP-40, 1mM PMSF, 25 µg/ml leupeptin, 25 µg/ml aprotitin, 1 mM benzamidine and 10 µg/ml trypsin inhibitor) and lysed by rotating for 15 to 30 minutes. Nuclei were removed by centrifugation at 15,000 g for 5 minutes and lysates were pre-cleared by incubating with either pre-immune serum or normal rabbit serum and IgG sorb (The Enzyme Center, Inc.) for 20 to 30 minutes followed by a 10 minute centrifugation at 15,000 g. Antibody pre-coupled to the protein A agarose beads (Pierce) was added to the clarified lysates and incubated for six to eight hours. Immunoprecipitates were washed three to four times with lysis buffer at room temperature, resuspended in SDS sample buffer and separated on SDS-polyacrylamide gels.

For the ³⁵S methionine-labelled precipitates, polyacrylamide gels (except those for V8 proteolytic mapping experiments) were fixed with 10% glacial acetic acid and 30% methanol for 30 minutes to one hour, enhanced by impregnating with autoradiography enhances (Du Pont) for 30 minutes and precipitated in water for 15 to 30 minutes. Enhanced gels were dried and exposed to X-ray films at -70°C. For Western-blotting, polypeptides were transferred to a nitrocellulose filter using a SDE Electroblotting System (Millipore) for 45 minutes at constant current of 400 mA. The filter was blocked for 1 to 3 hours with TBST (20 mM Tris-HCl, pH 7.5, 137 mM NaCl, 0.1% Tween-20) containing 5% dry milk, incubated with primary antibody for 4 hours to overnight in TBST containing 5% dry milk and washed 4 times, 10 minutes each time, with TBST. Appropriate secondary antibody (1:10,000 dilution of either horseradish peroxidase linked sheet anti-mouse Ig or donkey anti-rabbit Ig, Amersham) were incubated with filters for one hour and specific proteins were detected using an enhanced chemiluminescence system (ECL, Amersham).

### (iv) Partial Proteolytic Peptide Mapping

Human cyclin D1, cyclin D2, cyclin D3, CDC2, CDK2, CDK3, CDK4, CDK5 and PCNA were subcloned into pBluescript vector (Stratagene) for *in vitro* translation with T7 RNA polymerase using a TNT coupled reticulocyte lysate system (Promega). Immunoprecipitation of [³⁵S] methionine-labelled lysates and SDS-polyacrylamide gel electrophoresis were the same as described above. Polyacrylamide gels were dried without prior fixation and enhanced treatment, exposed to Fuji image plates and visualized on Fuji bio-imaging analyzer BAS2000. Appropriate protein bands were excised from the gels using image printout as template, in-gel partially digested with various amount of S. aureus V8 protease according to (Cleveland, *et al., J. Biol. Chem. 252*:1102-1106 (1977)) and (Harlow and Lane, *Antibodies: a laboratory manual*, Cold Spring Harbor Laboratory Press, NY (1988)), separated on a 17.5% SDS-PAGE. Gels were dried and exposed to a X-ray film for 2 weeks, or analyzed on a Fuji image analyzer BAS2000.

### EXAMPLE 2

### Demonstration of Selective Subunit Rearrangement of Cell Cycle Complexes In Association With Cellular Transformation by a DNA Tumor Virus or Its Oncogenic Product

### (i) Cellular Transformation With DNA Tumor Virus SV40 Is Associated With Subunits Rearrangement of Cell Cycle Complexes

Preparation of [³⁵S] methionine-labelled cell lysates and polyacrylamide gel electrophoresis were as described above, as well as described in PCT Publication No. WO92/20796. Cell lysates were prepared from either human normal diploid fibroblast cells WI38 or DNA tumor virus SV40 transformed WI38 cells, VA13. Cell lysates were immunoprecipitated with antibodies against each cell cycle gene products.

### (ii) Subunit Rearrangements of Cell Cycle Complexes In Two Different Pair Cell Lines

Methods for preparation of cell lysates are the same as described above. Two different pair cell lines were used in these experiments. HSF43 is a normal human diploid fibroblast cell line and CT10 (full name CT10-2C-T1) is a derivative of HSF43 transformed by SV40 large tumor antigen. CV-1 is an African green monkey kidney cell line and COS-1 is a derivative of CV-1 transformed by SV40.

### (iii) Cellular Transformation by DNA Tumor Virus SV40 Is Associated With Rearrangement of PCNA Subunit of Cell Cycle Complexes

Preparation of cell lysate, electrophoresis, and Western blotting conditions are the same as described above. Normal human diploid fibroblast cell lines and their SV40 transformed cell lines are described above. Immunoprecipitates derived from each antibody were separated on polyacrylamide gels and blotted with anti-PCNA antibody.

### (iv) Cellular Transformation by DNA Tumor Virus SV40 Is Associated With Rearrangement of CDK4 Subunit of Cell Cycle Complexes

Preparation of cell lysate, electrophoresis, and Western blotting conditions are the same as previously described. Normal human diploid fibroblast cell lines and their SV40 transformed cell lines are described above. Immunoprecipitates derived from each antibody were separated on polyacrylamide gels and blotted with anti-CDK4 antibody.

### Example 3

### Cloning of p16^{INK4}, an inhibitor of CDK4 activity

### (i) Cloning of p16^{INK4} using the two hybrid assay

Saccharomyces cerevisiae YPB2 cells were transformed simultaneously with a plasmid containing, a GAL4db-p16INK4 fusion and with a plasmid containing, respectively, the GAL4ad fused to cdc2 (CDK1), CDK2, CDK4, CDK5, PCNA (proliferating cell nuclear antigen), and the fission yeast kinase Snf 1. After growing cells in medium selective for both plasmids (minus tryptophan and minus leucine), two colonies were picked randomly and were streaked in plates that either contained or lacked histidine. The ability to grow in the absence of histidine depends on the expression of the HIS3 gene that is under a GAL4-responsive promoter and, therefore, indicates that a functional GAL4 activator has been reconstituted through the interaction of p16INK4 with the corresponding target protein.

### (ii) Interaction of p16^{INK4} CDKs

Purified bacterially-produced GSTp16INK4 fusion protein was mixed with (³⁵S)-labeled *in vitro* translated cdc2, CDK2, CDK4 and CDK5. Mixtures contained 0.5 µg of purified GST-p16INK4 and an equivalent amount of *in vitro* translated protein (between 0.5 to 5 ,µl; TNT Promega) in a final volume of 200 µl of a buffer containing 50 mM Tris-HCl pH 8, 120 mM NaCl and 0.5% Nonidet P-40. After 1 h at 4°C, 15 µl of glutathione-agarose beads were added and incubation was resumed for an additional hour. Beads were recovered by centrifugation, washed 4 times with the incubation buffer, and mixed with standard protein-gel loading buffer. Samples were loaded into a 15% poly-acryllamide gel and (³⁵S)-labeled proteins were detected by fluorography. The GSTp16INK4 fusion protein was overexpressed in the pGEX-KG vector and purified by standard techniques. The *in vitro* translation templates were derived from the pBluescript vector (Stratagene).

### (iii) Proteolytic mapping of p16^{INK4}

The *in vitro* translated (³⁵S)-labeled p16INK4 (TNT Promega) was obtained using the p16INK4 cDNA cloned into pBluescript vector (Stratagene) as a template, and the CDK4-associated p16 protein was co-immunoprecipitated with an anti-CDK4 serum from metabolically (³⁵S)-labeled HeLa cells lysates. Partial proteolysis was done over the corresponding gel slices after extensive equilibration in a buffer and digestion was accomplished by addition of NCS at different concentrations. The products were run in a 17.5% polyacrilamide gel and detected in a phosphoimager Fujix 2000.

### (iv) Detecting the effects of p16^{INK4} on CDK4-cyclin D complexes

Baculovirus-infected insect cells overexpressing p16INK4, CDK4, cyclin D1, or both CDK4 and cyclin D1 together were metabolically (³⁵S)-labeled. The different incubation mixtures were composed by extracts containing p16INK4, CDK4, cyclin D1 and both CDK4 and cyclin D1, and were immunoprecipitated with anti-p16INK4 serum, anti-CDK4 serum without any previous preincubation, and anti-CDK serum preincubated with the peptide originally used to raise the antiserum and anti-cyclin D1 serum. Immunoprecipitates were then analyzed by SDS-PAGE.

## Claims

1. A method of identifying a transformed or abnormally proliferating cell, comprising:
(A) determining, in a test cell(s), the subunit composition of a complex comprising a protein having a molecular weight of about 16 kDa, 19 kDa or 21 kDa and either a cyclin dependent kinase (cdk), a cyclin, or both a cdk and a cyclin, wherein the protein is predominantly a protein having an apparent molecular weight of about 21KDa in a normal cell, or is predominantly a protein having an apparent molecular weight of about 19KDa or about 16KDa in a transformed or abnormally proliferating cell, and
(B) comparing the subunit composition of step (A) with the subunit composition of a like complex found in a normal cell, wherein an alteration in the subunit composition is indicative of transformation or abnormal proliferation of the test cell.

2. The method of claim 1 wherein (A) comprises ascertaining the level of a protein having an apparent molecular weight of 21 KDa complexed with a cdk, a cyclin or both a cdk and a cyclin and wherein in (B) a decrease in the level of said 21 kDa protein complexed with a cdk, a cyclin or both a cdk and a cyclin is indicative of transformation or abnormal proliferation of the test cell(s).

3. The method of claim 1 wherein (A) comprises ascertaining the level of a protein having an apparent molecular weight of 16 kDa complexed with a cdk and wherein in (B) an increase in the level of said 16 kDa protein complexed with a cdk in indicative of transformation or abnormal proliferation of the test cell(s).

4. The method of claim 1 wherein (A) comprises ascertaining the level of a protein having an apparent molecular weight of 19 kDa complexed with a cyclin and wherein in (B) an increase in the level of said 19 kDa protein complexed with a cyclin is indicative of transformation or abnormal proliferation of the test cell(s).

5. The method of any preceding claim, wherein gel electrophoresis analysis and/or an immunoassay is used to ascertain the subunit composition.

6. The method of claim 1, wherein the cyclin is a D-type cyclin or an A-type cyclin.

7. The method of claim 1, wherein the cdk is cdk4.

8. The method of claim 1, wherein the cyclin is cyclin A.

9. The method of any preceding claim, which method is an *in vitro* diagnostic method for assessing a disease state or the probability of developing a condition associated with transformation or abnormal proliferation of cells.

10. A purified and/or recombinant polypeptide comprising an amino acid sequence of a polypeptide having an apparent molecular mass of about 15 kDa to 17 kDa, wherein said polypeptide inhibits a cyclin dependent kinase and is encoded by a nucleic acid which specifically hybridizes to the nucleic acid sequence of SEQ ID No. 3.

11. A polypeptide according to claim 10 which is a fusion protein or an immunogenic portion of the protein of SEQ ID No. 4.

12. The polypeptide of claim 10, wherein the polypeptide comprises the amino acid sequence of SEQ ID No.4.

13. The polypeptide of claim 10, wherein the polypeptide has a molecular weight of 16 kDa.

14. The polypeptide of claim 10, wherein the polypeptide is a recombinant polypeptide.

15. The polypeptide of claim 10, wherein the polypeptide is specifically reactive with an antibody selective for the protein of SEQ ID No.4.

16. An isolated nucleic acid encoding the polypeptide of claim 10.

17. A nucleic acid according to claim 16 encoding the protein of SEQ ID No.4.

18. A nucleic acid according to claim 16 comprising the nucleotide sequence of SEQ ID No.3.

19. An isolated nucleic acid (sense or antisense) which specifically hybridizes to the nucleic acid sequence of SEQ ID No. 3 and selectively detects a gene encoding a 16 kDa protein which binds to cyclin dependent kinase 4 (cdk4).

20. The nucleic acid of claim 19, wherein the nucleic acid is an antisense oligonucleotide which hybridizes to the gene and inhibits expression of said protein.

21. A diagnostic test kit for identifying transformed cells comprising:
(a) a nucleic acid probe (sense or antisense) which specifically hybridizes to the nucleic acid sequence of SEQ ID No. 3 and selectively detects a nucleic acid encoding a 16 kDa protein which binds to cyclin dependent kinase 4 (cdk4), which probe is for measuring, in a sample of cells isolated from a patient, the presence or absence of a nucleic acid encoding said 16 kDa protein; and/or
(b) an antibody specific for the 16 kDa protein for measuring, in a sample of cells isolated from a patient, an amount of said protein.

22. A kit according to claim 21 wherein the antibody is specific for said polypeptide which has an amino acid sequence of SEQ ID No. 4.

23. The nucleic acid of any of claims 16 to 20 or the kit of claims 21 or 22, wherein the nucleic acid, the probe or the antibody is labelled with a detectable label selected from the group consisting of: an enzyme, an enzyme substrate, a coenzyme, an enzyme inhibitor, a fluorescent marker, a chromophore, a luminescent marker, a specifically bindable ligand and a radioisotope.

24. The nucleic acid or the kit of any of claims 19 to 21 or 23, wherein the nucleic acid or probe hybridizes to an mRNA transcript encoding said protein.

25. The nucleic acid or the kit of any of claims 16 to 21 or 23, wherein the nucleic acid or probe is approximately 75 to 150 nucleotides in length.

26. A recombinant expression vector comprising a nucleic acid encoding said polypeptide of claim 4 or the nucleic acid of claim 16 or claim 19.

27. A preparation comprising a fusion protein of any of claims 10 to 15, wherein the fusion protein is greater than 90% pure.

28. An assay method for identifying an agent which alters the subunit composition of a cyclin-dependent kinase complex, comprising
i. providing a cyclin-dependent kinase (cdk) and the polypeptide of any of claims 10 to 15, under conditions wherein the cdk and said polypeptide can form a complex;
ii. contacting the mixture with a candidate agent;
iii. detecting an amount of polypeptide as part of the complex formed;
iv. comparing the amount of polypeptide present in the complex in the presence of the candidate agent to the amount of polypeptide present in the complex in the absence of the candidate agent,
wherein a change in the amount of polypeptide present in the complex in the presence of the candidate agent, relative to the absence of candidate agent, is indicative of an agent which alters the subunit composition of a cyclin-dependent kinase complex.

29. The method of claim 28, wherein the mixture is a cell lysate or an in vitro reconstituted protein preparation.

30. The method of claim 1 or 29, wherein the cell(s) is obtained from a tissue sample or biological sample.

31. A preparation comprising a polypeptide of any of claims 10 to 15, and cyclin dependent kinase cdk4 for use in the assay method of claim 28.

32. A method for detecting transformation of a mammalian cell by detecting altered expression of a gene encoding a protein having a molecular weight of 15 kDa to 17 kDa which is an inhibitor of cdk4, which method comprises determining, in a test sample of mammalian cells, the presence or absence of nucleic acid which specifically hybridises to the nucleic acid of SEQ ID No. 3, the presence of said nucleic acid indicating transformation of the cells.

33. The method of claim 32, wherein nucleic acid hybridization is used to determine the level of mRNA transcripts encoding the 15 kDa to 17 kDa protein inhibitor of cyclin dependent kinase.

34. An antibody which is specifically immunoreactive with the protein of SEQ ID No. 4.

## Patentansprüche

1. Verfahren zum Identifizieren einer transformierten oder anormal proliferierenden Zelle, umfassend:
(A) Bestimmen, in (einer) Testzelle(n), der Untereinheitzusammensetzung eines Komplexes, umfassend ein Protein mit einem Molekulargewicht von etwa 16 kDa, 19 kDa oder 21 kDa und entweder eine cyclinabhängige Kinase (cdk), ein Cyclin oder sowohl eine cdk als auch ein Cyclin, wobei das Protein überwiegend ein Protein mit einem scheinbaren Molekulargewicht von etwa 21 kDa in einer normalen Zelle ist oder überwiegend ein Protein mit einem scheinbaren Molekulargewicht von etwa 19 kDa oder etwa 16 kDa in einer transformierten oder anormal proliferierenden Zelle ist, und
(B) Vergleichen der Untereinheitzusammensetzung von Schritt (A) mit der Untereinheitzusammensetzung eines ähnlichen Komplexes, gefunden in einer normalen Zelle, wobei eine Veränderung in der Untereinheitzusammensetzung ein Anzeichen von Transformation oder anormaler Proliferation der Testzelle ist

2. Verfahren nach Anspruch 1, wobei (A) Ermitteln des Gehalts an einem Protein mit einem scheinbaren Molekulargewicht von 21 kDa, komplexiert mit einer cdk, einem Cyclin oder sowohl einer cdk als auch einem Cyclin, umfaßt und wobei in (B) eine Abnahme in dem Gehalt an dem 21-kDa-Protein, komplexiert mit einer cdk, einem Cyclin oder sowohl einer cdk als auch einem Cyclin, ein Anzeichen von Transformation oder anormaler Proliferation der Testzelle(n) ist

3. Verfahren nach Anspruch 1, wobei (A) Ermitteln des Gehalts an einem Protein mit einem scheinbaren Molekulargewicht von 16 kDa, komplexiert mit einer cdk, umfaßt und wobei in (B) eine Zunahme in dem Gehalt an dem 16-kDa-Protein, komplexiert mit einer cdk, ein Anzeichen von Transformation oder anormaler Proliferation der Testzelle(n) ist.

4. Verfahren nach Anspruch 1, wobei (A) Ermitteln des Gehalts an einem Protein mit einem scheinbaren Molekulargewicht von 19 kDa, komplexiert mit einem Cyclin, umfaßt und wobei in (B) eine Zunahme in dem Gehalt an dem 19-kDa-Protein, komplexiert mit einem Cyclin, ein Anzeichen von Transformation oder anormaler Proliferation der Testzelle(n) ist.

5. Verfahren nach einem vorhergehenden Anspruch, wobei Gelelektrophorese-Analyse und/oder ein Immunoassay verwendet werden, um die Untereinheitzusammensetzung zu ermitteln.

6. Verfahren nach Anspruch 1, wobei das Cyclin ein D-Typ-Cyclin oder ein A-Typ-Cyclin ist.

7. Verfahren nach Anspruch 1, wobei die cdk cdk4 ist.

8. Verfahren nach Anspruch 1, wobei das Cyclin Cyclin A ist.

9. Verfahren nach einem vorhergehenden Anspruch, welches Verfahren ein diagnostisches in-vitro-Verfahren zur Beurteilung eines Krankheitszustands oder der Wahrscheinlichkeit der Entwicklung eines Leidens, verbunden mit Transformation oder anormaler Proliferation von Zellen, ist.

10. Gereinigtes und/oder rekombinantes Polypeptid, umfassend eine Aminosäuresequenz eines Polypeptids mit einer scheinbaren Molekülmasse von etwa 15 kDa bis 17 kDa, wobei das Polypeptid eine cyclinabhängige Kinase hemmt und durch eine Nucleinsäure kodiert wird, welche spezifisch mit der Nucleinsäuresequenz der SEQ ID No. 3 hybridisiert.

11. Polypeptid nach Anspruch 10, welches ein Fusionsprotein oder ein immunogener Anteil des Proteins der SEQ ID No. 4 ist.

12. Polypeptid nach Anspruch 10, wobei das Polypeptid die Aminosäuresequenz der SEQ ID No. 4 umfaßt.

13. Polypeptid nach Anspruch 10, wobei das Polypeptid ein Molekulargewicht von 16 kDa hat.

14. Polypeptid nach Anspruch 10, wobei das Polypeptid ein rekombinantes Polypeptid ist.

15. Polypeptid nach Anspruch 10, wobei das Polypeptid spezifisch reaktiv mit einem Antikörper, selektiv für das Protein der SEQ ID No. 4, ist

16. Isolierte Nucleinsäure, kodierend das Polypeptid nach Anspruch 10.

17. Nucleinsäure nach Anspruch 16, kodierend das Protein der SEQ ID No. 4.

18. Nucleinsäure nach Anspruch 16, umfassend die Nucleotidsequenz der SEQ ID No.3.

19. Isolierte Nucleinsäure (Sense oder Antisense), welche spezifisch mit der Nucleinsäuresequenz der SEQ ID No. 3 hybridisiert und selektiv ein Gen, kodierend ein 16-kDa-Protein, nachweist, welches an cyclinabhängige Kinase 4 (cdk4) bindet.

20. Nucleinsäure nach Anspruch 19, wobei die Nucleinsäure ein Antisense-Oligonucleotid ist, welches mit dem Gen hybridisiert und die Expression des Proteins hemmt.

21. Diagnostisches Test-Kit zum Identifizieren transformierter Zellen, umfassend:
(a) eine Nucleinsäuresonde (Sense oder Antisense), welche spezifisch mit der Nucleinsäuresequenz der SEQ ID No. 3 hybridisiert und selektiv eine Nucleinsäure, kodierend ein 16-kDa-Protein, welches an cyclinabhängige Kinase 4 (cdk4) bindet, nachweist, welche Sonde zurn Messen, in einer Probe von Zellen, isoliert von einem Patienten, der Anwesenheit oder Abwesenheit einer Nucleinsäure, kodierend das 16-kDa-Protein, ist; und/oder
(b) einen Antikörper, spezifisch für das 16-kDa-Protein, zum Messen, in einer Probe von Zellen, isoliert von einem Patienten, einer Menge des Proteins.

22. Kit nach Anspruch 21, wobei der Antikörper spezifisch für das Polypeptid ist, welches eine Aminosäuresequenz der SEQ ID No, 4 aufweist.

23. Nucleinsäure nach einem der Ansprüche 16 bis 20 oder das Kit nach den Ansprüchen 21 oder 22, wobei die Nucleinsäure, die Sonde oder der Antikörper mit einer nachweisbaren Markierung, ausgewählt aus der Gruppe, bestehend aus: einem Enzym, einem Enzyinsubstrat, einem Coenzym, einem Enzyminhibitor, einer Fluoreszenzmarkierung, einem Chromophor, einer Lumineszenzmariderung, einem spezifisch bindungsfähigen Liganden und einem Radioisotop, markiert ist.

24. Nucleinsäure oder das Kit nach einem der Ansprüche 19 bis 21 oder 23, wobei die Nucleinsäure oder Sonde mit einem mRNA-Transkript, kodierend das Protein, hybridisiert.

25. Nucleinsäure oder das Kit nach einem der Ansprüche 16 bis 21 oder 23, wobei die Nucleinsäure oder Sonde eine Länge von ungefähr 75 bis 150 Nucleotiden hat

26. Rekombinanter Expressionsvektor, umfassend eine Nucleinsäure, kodierend das Polypeptid nach Anspruch 4 oder die Nucleinsäure nach Anspruch 16 oder Anspruch 19.

27. Zubereitung, umfassend ein Fusionsprotein nach einem der Ansprüche 10 bis 15, wobei das Fusionsprotein zu mehr als 90% rein ist.

28. Testverfahren zum Identifizieren eines Mittels, welches die Untereinheitzusammensetzung eines cyclinabhängigen Kinasekomplexes verändert, umfassend
i. Bereitstellen einer cyclinabhängigen Kinase (cdk) und des Polypeptids nach einem der Ansprüche 10 bis 15 unter Bedingungen, unter denen die cdk und das Polypeptid einen Komplex bilden können;
ii. inkontaktbringen des Gemisches mit einem in Frage kommenden Mittel;
iii. Nachweisen einer Menge von Polypeptid als Teil des gebildeten Komplexes;
iv. Vergleichen der Menge des Polypeptids, vorhanden in dem Komplex in Anwesenheit des in Frage kommenden Mittels, mit der Menge des Polypeptids, vorhanden in dem Komplex in Abwesenheit des in Frage kommenden Mittels,
wobei eine Änderung in der Menge des Polypeptids, vorhanden in dem Komplex in Anwesenheit des in Frage kommenden Mittels relativ zu der in Abwesenheit des in Frage kommenden Mittels, ein Anzeichen eines Mittels ist, welches die Untereinheitzusammensetzung eines cyclinabhängigen Kinasekomplexes verändert.

29. Verfahren nach Anspruch 28, wobei das Gemisch ein Zell-Lysat oder eine invitro-rekonstituierte Proteinzubereitung ist.

30. Verfahren nach Anspruch 1 oder 29, wobei die Zelle(n) aus einer Gewebeprobe oder biologischen Probe erhalten wird (werden).

31. Zubereitung, umfassend ein Polypeptid nach einem der Ansprüche 10 bis 15 und eine cyclinabhängige Kinase cdk4 zur Verwendung in dem Testverfahren nach Anspruch 28.

32. Verfahren zum Nachweisen der Transformation einer Säugetierzelle durch Nachweisen der veränderten Expression eines Gens, kodierend ein Protein mit einem Molekulargewicht von 15 kDa bis 17 KDa, welches ein Inhibitor von cdk4 ist, welches Verfahren Bestimmen, in einer Testprobe von Säugetierzellen, der Anwesenheit oder Abwesenheit von Nucleinsäure, welche spezifisch mit der Nucleinsäure der SEQ ID No. 3 hybridisiert, umfaßt, wobei die Anwesenheit der Nucleinsäure Transformation der Zellen anzeigt.

33. Verfahren nach Anspruch 32, wobei die Nucleinsäurehybridisierung verwendet wird, um den Gehalt an mRNA-Transkripten, kodierend den 15-kDa- bis 17-kDa-Proteininhibitor von cyclinabhängiger Kinase, zu bestimmen.

34. Antikörper, welcher spezifisch immunoreaktiv mit dem Protein der SEQ ID No. 4 ist.

## Revendications

1. Procédé d'identification d'une cellule transformée ou à prolifération anormale, comprenant les étapes ci-dessous:
(A) détermination, dans une (des) cellule(s) de test, de la composition de sous-unité d'un complexe comprenant une protéine ayant un poids moléculaire de l'ordre de 16 kDa, 19 kDa ou 21 kDa et une kinase dépendant de la cycline (cdk), une cycline ou une cdk et une cycline, la protéine étant pour l'essentiel une protéine ayant un poids moléculaire apparent de l'ordre de 23 kDa dans une cellule normale, ou étant pour l'essentiel une protéine ayant un poids moléculaire apparent de l'ordre de 19 kDa ou de l'ordre de 16 kDa dans une cellule transformée ou à prolifération anormale, et
(B) comparaison de la composition de sous-unité de l'étape (A) avec la composition de sous-unité d'un complexe similaire présent dans une cellule normale, un changement dans la composition de sous-unité indiquant une transformation ou une prolifération anormale de la cellule de test.

2. Procédé selon la revendication 1, dans lequel l'étape (A) comprend la détermination du niveau d'une protéine ayant un poids moléculaire apparent de 21 kDa formant un complexe avec une cdk, une cycline ou une cdk et une cycline, une réduction dans l'étape (B) du niveau de ladite protéine de 21 kDa formant un complexe avec une cdk, une cycline ou une cdk et une cycline indiquant une transformation ou une prolifération anormale de la (des) cellule(s) de test.

3. Procédé selon la revendication A, dans lequel l'étape (A) comprend la détermination du niveau d'une protéine ayant un poids moléculaire apparent de 16 kDa formant un complexe avec une cdk, un accroissement lors de l'étape (B) du niveau de ladite protéine de 16 kDa formant un complexe avec une cdk indiquant une transformation ou une prolifération anormale de la (des) cellule(s) de test,

4. Procédé selon la revendication 1, dans lequel l'étape (A) comprend la détermination du niveau d'une protéine ayant un poids moléculaire apparent de 19 kDa formant un complexe avec une cycline, un accroissement lors de l'étape (B) du niveau de ladite protéine de 19 kDa formant un complexe avec une cycline indiquant une transformation ou une prolifération anormale de la (des) cellule(s) de test

5. Procédé selon l'une quelconque des revendications précédentes, dans lequel une analyse à électrophorèse sur gel et/ou une analyse immunologique est utilisée pour déterminer la composition de sous-unité,

6. Procédé selon la revendication 1, dans lequel la cycline est une cycline de type D ou une cycline de type A.

7. Procédé selon la revendication 1, dans lequel la cdk est une cdk4.

8. Procédé selon la revendication 1, dans lequel la cycline est une cycline A.

9. Procédé selon l'une quelconque des revendications précédentes, le procédé étant un procédé de diagnostic in vitro destiné à évaluer un état diagnostique ou la probabilité du développement d'un état associé à une transformation ou une prolifération anormale des cellules.

10. Polypeptide purifié et/ou recombinant comprenant une séquence d'acides aminés d'un polypeptide ayant un poids moléculaire apparent compris entre environ 15 kDa et environ 17 kDa, ledit polypeptide inhibant une kinase dépendant de la cycline et étant codé par un acide nucléique hybridé spécifiquement avec la séquence d'acides nucléiques de SEQ ID no.3.

11. Polypeptide selon la revendication 10, constituant une protéine de fusion ou une partie immunogène de la protéine de SEQ ID no.4.

12. Polypeptide selon la revendication 10, dans lequel le polypeptide comprend la séquence d'aminoacide de SEQ ID no. 4.

13. Polypeptide selon la revendication 10, dans lequel le polypeptide a un poids moléculaire de 16 kDa.

14. Polypeptide selon la revendication 10, dans lequel le polypeptide est un polypeptide recombinant.

15. Polypeptide selon la revendication 10, dans lequel le polypeptide réagit spécifiquement avec un anticorps sélectif de la protéine de SEQ ID no.4.

16. Acide nucléique isolé codant le polypeptide selon la revendication 10.

17. Acide nucléique selon la revendication 16, codant la protéine de SEQ ID no. 4.

18. Acide nucléique selon la revendication 16, comprenant la séquence nucléotide de SEQ ID no. 3.

19. Acide nucléique isolé (sens ou anti-sens) hybridé spécifiquement avec la séquence d'acides nucléiques de SEQ ID no. 3 et détectant sélectivement un gène codant une protéine de 16 kDa se liant à la kinase dépendant de la cycline 4 (cdk4).

20. Acide nucléique selon la revendication 19, dans lequel l'acide nucléique est un oligonucléotide asti-sens hybridé avec le gène et inhibant l'expression de ladite protéine.

21. Kit de test diagnostique pour identifier des cellules transformées, comprenant
(a) une sonde d'acide nucléique (sens ou anti-sens) hybridée spécifiquement avec la séquence d'acide nucléique de SEQ ID no. 3 et détectant sélectivement un acide nucléique codant une protéine de 16 kDa se liant à la kinase dépendant de la cycline 4 (cdk4), cette sonde servant à mesurer, dans un échantillon de cellules isolées d'un patient, la présence ou l'absence d'un acide nucléique codant ladite protéine de 16 kDa; et/ou
(b) un anticorps spécifique de la protéine de 16 kDa pour mesurer, dans un échantillon de cellules isolées d'un patient, une quantité de ladite protéine.

22. Kit selon la revendication 21, dans lequel l'anticorps est spécifique dudit polypeptide ayant une séquence d'acide aniné de SEQ ID no. 4.

23. Acide nucléique selon l'une quelconque des revendications 16 à 20 ou kit selon les revendications 21 ou 22, dans lequel l'acide nucléique, la sonde ou l'anticorps est marqué avec un marqueur détectable sélectionné dans le groupe constitué de: une enzyme, un substrat d'enzyme, une coenzyme, un inhibiteur d'enzyme, un marqueur fluorescent, un chromophore, un marqueur luminescent, un ligand à liaison spécifique et un radio-isotope.

24. Acide nucléique ou kit selon l'une quelconque des revendications 19 à 21 ou 23, dans lequel l'acide nucléique ou la sonde est hybridée avec un transcrit d'ARNm codant ladite protéine.

25. Acide nucléique ou kit selon l'une quelconque des revendications 16 à 21 ou 23, dans lequel l'acide nucléique ou la sonde a une longueur comprise entre environ 75 et 150 nucléotides.

26. Vecteur d'expression recombinant comprenant un acide nucléique codant ledit polypeptide selon la revendication 4 ou l'acide nucléique selon les revendications 16 ou 19.

27. Préparation comprenant une protéine de fusion selon l'une quelconque des revendications 10 à 15, dans laquelle la protéine de fusion a une pureté supérieure à 90%.

28. Procédé d'analyse destiné à identifier un agent modifiant la composition de sous-unité d'un complexe de kinase dépendant de la cycline, comprenant les étapes ci-dessous:
i. fourniture d'une kinase dépendant de la cycline (cdk) et du polypeptide selon l'une quelconque des revendications 10 à 15, dans des conditions dans lesquelles la cdk et ledit polypeptide peuvent former un complexe;
ii. mise en contact du mélange avec un agent à analyser:
iii. détection d'une quantité de polypeptide comme partie du complexe formé;
iv. comparaison de la quantité de polypeptide présent dans le complexe en présence de l'agent à analyser avec la quantité de polypeptide présente dans le complexe en cas d'absence de l'agent à analyser,
un changement de la quantité de polypeptide présente dans le complexe en cas de présence de l'agent à analyser par rapport à celle présente en cas d'absence de l'agent à analyser indiquant la présence d'un agent modifiant la composition de sous-unité du complexe de kinase dépendant de la cycline.

29. Procédé selon la revendication 28, dans lequel le mélange est un lysat cellulaire ou une préparation de protéine reconstituées in vitro.

30. Procédé selon les revendications 1 à 29, dans lequel la (les) cellule(s) est (sont) obtenue(s) à partir d'un échantillon tissulaire ou d'un échantillon biologique.

31. Préparation comprenant un polypeptide selon l'une quelconque des revendications 10 à 15, et une kinase dépendant de la cycline cdk4 destinée à être utilisée dans le procédé d'analyse selon la revendication 28.

32. Procédé de détection de la transformation d'une cellule mammalienne par détection de l'expression modifiée d'un gène codant une protéine ayant un poids moléculaire compris entre 15 kDa et 17 kDa, constituant un inhibiteur de la cdk4, le procédé comprenant l'étape de détermination, dans un échantillon de test de cellules mammaliennes, de la présence ou de l'absence d'acide nucléique hybridé spécifiquement avec l'acide nucléique de SEQ ID no. 3, la présence dudit acide nucléique indiquant la transformation des cellules.

33. Procédé selon la revendication 32, dans lequel l'hybridation de l'acide nucléique sert à déterminer le niveau des transcrits d'ARNm codant la protéine de 15 kDa à 17 kDa inhibant une kinase dépendant de la cycline.

34. Anticorps spécifiquement immunoréactif avec la protéine de SEQ ID no. 4.
